# EUROPÄISCHE PATENTSCHRIFT

(11) **EP 0 905 139 B1**
(45) Veröffentlichungstag und Bekanntmachung des Hinweises auf die Patenterteilung: **21.01.2009**
(21) Anmeldenummer: 98117660.5
(22) Anmeldetag: 17.09.1998
(51) Int. Cl.: C07K 5/023, C07K 5/107, A61K 38/06, A61K 38/07, A61P 29/00

(54) **5-Ring-Heterocyclen, ihre Herstellung, ihre Verwendung als Inhibitoren der Leukozytenadhäsion und sie enthaltende pharmazeutische Präparate**
5 ring heterocyles, process for preparation, use as inhibitors of leukocyte adhesion and pharmaceutical preparations comprising them
Hétérocycles à cinq chainons, procédé pour leur préparation, leur utilisation à titre d'inhibiteurs d'adhésion des leucocytes et préparations pharmaceutiques les contenant

(30) Priorität: 23.09.1997 DE 19741873
(43) Veröffentlichungstag der Anmeldung: 31.03.1999
(73) Patentinhaber: Sanofi-Aventis Deutschland GmbH, 65929 Frankfurt am Main (DE)
(72) Erfinder: Wehner, Volkmar Dr., 97657 Sandberg (DE); Stilz, Hans Ulrich Dr., 65929 Frankfurt (DE); Schmidt, Wolfgang Dr., 65929 Frankfurt (DE); Seiffge, Dirk Dr., 55246 Mainz-Kostheim (DE)

(56) Entgegenhaltungen:
- EP-A- 0 530 505
- EP-A- 0 580 008
- EP-A- 0 584 694
- EP-A- 0 842 943
- EP-A- 0 842 944
- EP-A- 0 842 945
- WO-A-95/14008
- WO-A-95/15973

## Beschreibung

Gegenstand der vorliegenden Erfindung sind Verbindungen der Formel I, 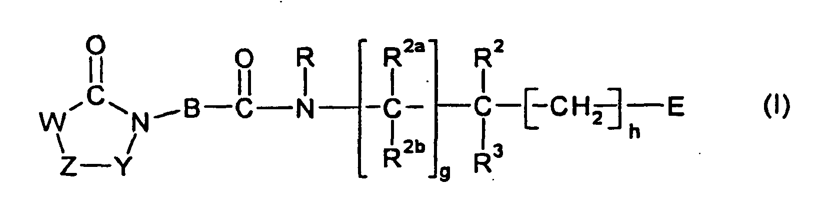 in der B, E, W, Y, Z, R, R², R^{2a}, R^{2b}, R³, g und h die unten angegebenen Bedeutungen haben. Die Verbindungen der Formel I sind wertvolle Arzneimittelwirkstoffe, die sich zum Beispiel zur Therapie und Prophylaxe von Entzündungserkrankungen, beispielsweise der rheumatoiden Arthritis, oder von allergischen Erkrankungen eignen. Die Verbindungen der Formel I sind Inhibitoren' der Adhäsion und Migration von Leukozyten und/oder Antagonisten des zur Gruppe der Integrine gehörenden Adhäsionsrezeptors VLA-4. Sie eignen sich generell zur Therapie oder Prophylaxe von Krankheiten, die durch ein unerwünschtes Ausmaß an Leukozytenadhäsion und/oder Leukozytenmigration verursacht werden oder damit verbunden sind, oder bei denen Zell-Zell- oder Zell-Matrix-Interaktionen eine Rolle spielen, die auf Wechselwirkungen von VLA-4-Rezeptoren mit ihren Liganden beruhen. Die Erfindung betrifft weiterhin Verfahren zur Herstellung der Verbindungen der Formel I, ihre Verwendung in der Therapie und Prophylaxe der genannten Krankheitszustände und pharmazeutische Präparate, die Verbindungen der Formel I enthalten.

Die Integrine sind eine Gruppe von Adhäsionsrezeptoren, die bei Zell-Zellbindenden und Zell-Extrazelluläre Matrix-bindenden Prozessen eine wesentliche Rolle spielen. Sie weisen eine αβ-heterodimere Struktur auf und zeigen eine weite zelluläre Verbreitung und ein hohes Maß an evolutiver Konservierung. Zu den Integrinen gehört zum Beispiel der Fibrinogen-Rezeptor auf Thrombozyten, der vor allem mit der RGD-Sequenz des Fibrinogens interagiert, oder der Vitronectin-Rezeptor auf Osteoclasten, der vor allem mit der RGD-Sequenz des Vitronectins oder des Osteopontins interagiert. Man teilt die Integrine in drei Großgruppen ein, die β2-Unterfamilie mit den Vertetem LFA-1, Mac-1 und p150/95, die insbesondere für Zell-Zell-Interaktionen des Immunsystems verantwortlich sind, und die Unterfamilien β1 und β3, deren Vertreter hauptsächlich die Zellanheftung an Komponenten der extrazellulären Matrix vermitteln (Ruoslahti, Annu. Rev. Biochem. 1988, 57, 375). Die Integrine der β1-Unterfamilie, auch VLA-Proteine (very late (activation) antigen) genannt, umfassen mindestens sechs Rezeptoren, die spezifisch mit Fibronektin, Kollagen und/oder Laminin als Liganden interagieren. Innerhalb der VLA-Familie ist das Integrin VLA-4 (α4β1) insofern untypisch, als es hauptsächlich auf lymphoide und myeloide Zellen begrenzt ist und bei diesen verantwortlich ist für Zell-Zell-Interaktionen mit einer Vielzahl von anderen Zellen. VLA-4 vermittelt zum Beispiel die Interaktion von T- und B-Lymphozyten mit dem Heparin II-Bindungsfragment von humanem Plasmafibronektin (FN). Die Bindung von VLA-4 mit dem Heparin 11-Bindungsfragment des Plasmafibronektins beruht vor allem auf einer Interaktion mit einer LDVP-Sequenz. Im Unterschied zum Fibrinogen-Rezeptor oder Vitronectin-Rezeptor ist VLA-4 kein typisches RGDbindendes Integrin (Kilger und Holzmann, J. Mol. Meth. 1995, 73, 347).

Die im Blut zirkulierenden Leukozyten zeigen normalerweise nur eine geringe Affinität zu den vaskulären endothelialen Zellen, die die Blutgefäße auskleiden. Zytokine, die von entzündetem Gewebe abgegeben werden, bewirken die Aktivierung von Endothelzellen und damit die Expression einer Vielzahl von Zelloberflächenantigenen. Diese umfassen zum Beispiel die Adhäsionsmoleküle ELAM-1 (endothelial cell adhesion molecule-1, auch als E-Selektin bezeichnet), das unter anderem Neutrophile bindet, ICAM-1 (intercellular adhesion molecule-1), das mit LFA-1 (leucocyte function-associated antigen 1) auf Leukozyten interagiert, und VCAM-1 (vascular cell adhesion molecule-1), das verschiedene Leukozyten, unter anderem Lymphozyten, bindet (Osbom et al., Cell 1989, 59, 1203). VCAM-1 ist, wie ICAM-1, ein Mitglied der Immunglobulin-Gen-Überfamilie. Identifiziert wurde VCAM-1 (zuerst bekannt als INCAM-110) als ein Adhäsionsmolekül, das auf endothelialen Zellen durch Entzündungs-Zytokine wie TNF und IL-1 und Lipopolysaccharide (LPS) induziert wird. Elices et al. (Cell 1990, 60, 577) zeigten, daß VLA-4 und VCAM-1 ein Rezeptor-Ligänd-Paar bilden, das die Anheftung von Lymphozyten an aktiviertes Endothel vermittelt. Die Bindung von VCAM-1 an VLA-4 erfolgt dabei nicht durch eine Interaktion des VLA-4 mit einer RGD-Sequenz, eine solche ist im VCAM-1-nicht enthalten (Bergelson et al., Current Biology 1995, 5, 615). VLA-4 tritt aber auch auf anderen Leukozyten auf, und über den VCAM-1/VLA-4-Adhäsionsmechanismus wird auch die Anheftung von anderen Leukozyten als Lymphozyten vermittelt. VLA-4 repräsentiert somit ein einzelnes Beispiel eines β1-Integrin-Rezeptors, der über die Liganden VCAM-1 bzw. Fibronektin sowohl bei Zell-Zell-Interaktionen als auch bei Zell-Extrazellulärer Matrix-Interaktionen eine wesentliche Rolle spielt.

Die Zytokin-induzierten Adhäsionsmoleküle spielen eine wichtige Rolle bei der Rekrutierung von Leukozyten in extravaskuläre Gewebebereiche. Leukozyten werden in entzündliche Gewebebereiche durch Zelladhäsionsmoleküle rekrutiert, die auf der Oberfläche von endothelialen Zellen exprimiert werden und als Liganden für Leukozyten-Zelloberflächen-Proteine oder -Proteinkomplexe (Rezeptoren) dienen (die Begriffe Ligand und Rezeptor können auch vice versa verwendet werden). Leukozyten aus dem Blut müssen zunächst an endotheliale Zellen anheften, bevor sie in das Synovium auswandern können. Da VCAM-1 an Zellen bindet, die das Integrin VLA-4 (α4β1) tragen, wie Eosinophile, T- und B-Lymphozyten, Monozyten oder auch Neutrophile, kommt ihm und dem VCAM-1/VLA-4-Mechanismus die Funktion zu, derartige Zellen aus dem Blutstrom in Infektionsgebiete und Entzündungsherde zu rekrutieren (Elices et al., Cell 1990, 60, 577; Osborn, Cell 1990, 62, 3; Issekutz et al., J. Exp. Med. 1996,183, 2175).

Der VCAM-1/VLA-4-Adhäsionsmechanismus wurde mit einer Reihe von physiologischen und pathologischen Prozessen in Verbindung gebracht. VCAM-1 wird außer von Zytokin-induziertem Endothel unter anderem noch von den folgenden Zellen exprimiert: Myoblasten, lymphoiden dendritischen Zellen und Gewebsmakrophagen, rheumatoidem Synovium, Zytokin-stimulierten Neuralzellen, parietalen Epithelzellen der Bowmans Kapsel, dem renalen Tubularepithel, entzündetem Gewebe bei Herz- und Nieren-Transplantat-Abstoßung und von Intestinalgewebe bei Graft versus host-Krankheit. VCAM-1 findet man auch exprimiert auf solchen Gewebearealen des arteriellen Endotheliums, die frühen arteriosklerotischen Plaques eines Kaninchenmodells entsprechen. Zusätzlich wird VCAM-1 auf follikulären dendritischen Zellen von humanen Lymphknoten exprimiert und findet sich auf Stromazellen des Knochenmarks, zum Beispiel in der Maus. Letzterer Befund weist auf eine Funktion von VCAM-1 in der B-Zell-Entwicklung hin. VLA-4 wird, außer auf Zellen haematopoetischen Ursprunges, auch zum Beispiel auf Melanoma-Zellinien gefunden, und der VCAM-1/VLA-4-Adhäsionsmechanismus wird mit der Metastasierung von solchen Tumoren in Verbindung gebracht (Rice et al., Science 1989, 246, 1303).

Die hauptsächliche Form, in der VCAM-1 in vivo auf endothelialen Zellen vorkommt und die die dominante Form in vivo ist, wird als VCAM-7D bezeichnet und trägt sieben Immunglobulin-Domänen. Die Domänen 4, 5 und 6 ähneln- in ihren Aminosäuresequenzen den Domänen 1, 2 und 3. Die vierte Domäne ist bei einer weiteren, aus sechs Domänen bestehenden Form, hier als VCAM-6D bezeichnet, durch alternatives Splicing entfernt. Auch VCAM-6D kann VLA-4-exprimierende Zellen binden.

Weitere Angaben zu VLA-4, VCAM-1, Integrinen und Adhäsionsproteinen finden sich zum Beispiel in den Artikeln von Kilger und Holzmann, J. Mol. Meth. 1995, 73, 347; Elices, Cell Adhesion in Human Disease, Wiley, Chichester 1995, S. 79; Kuijpers, Springer Semin. Immunopathol. 1995,16, 379.

Aufgrund der Rolle des VCAM-1/VLA-4-Mechanismus bei Zelladhäsionsprozessen, die von Bedeutung zum Beispiel bei Infektionen, Entzündungen oder Atherosklerose sind, wurde versucht, durch Eingriffe in diese Adhäsionsprozesse Krankheiten zu bekämpfen, insbesondere zum Beispiel Entzündungen (Osborn et al., Cell 1989, 59, 1203). Eine Methode hierzu ist die Verwendung von monoklonalen Antikörpern, die gegen VLA-4 gerichtet sind. Derartige monoklonale Antikörper (mAK), die als VLA-4-Antagonisten die Interaktion zwischen VCAM-1 und VLA-4 blockieren, sind bekannt. So inhibieren zum Beispiel die anti-VLA-4 mAK HP2/1 und HP1/3 die Anheftung von VLA-4 exprimierenden Ramos-Zellen (B-Zell-ähnlichen Zellen) an humane Nabelschnurendothelzellen und an VCAM-1-transfizierte COS-Zellen. Ebenso inhibiert der anti-VCAM-1 mAK 4B9 die Adhäsion von Ramos-Zellen, Jurkat-Zellen (T-Zell-ähnlichen Zellen) und HL60-Zellen (Granulozyten-ähnlichen Zellen) an COS-Zellen transfiziert mit genetischen Konstrukten, die veranlassen, daß VCAM-6D und VCAM-7D exprimiert werden. In vitro-Daten mit Antikörpern, die gegen die α4-Untereinheit von VLA-4 gerichtet sind, zeigen, daß die Anheftung von Lymphozyten an synoviale Endothelzellen blockiert wird, eine Adhäsion, die bei der rheumatoiden Arthritis eine Rolle spielt (van Dinther-Janssen et al., J. lmmunol. 1991, 147, 4207).

In vivo-Versuche haben gezeigt, daß eine experimentelle autoimmune Enzephalomyelitis durch anti-α4 mAK gehemmt werden kann. Die Wanderung von Leukozyten in einen Entzündungsherd wird ebenfalls durch einen monoklonalen Antikörper gegen die α4-Kette von VLA-4 blockiert. Die Beeinflussung des VLA-4-abhängigen Adhäsionsmechanismus mit Antikörpern wurde auch in einem Asthma-Modell untersucht, um die Rolle von VLA-4 bei der Rekrutierung von Leukozyten in entzündetes Lungengewebe zu untersuchen (USSN 07/821,768: EP-A-626 861). Die Gabe von anti-VLA-4-Antikörpem inhibierte die Spätphasenreaktion und die Atemwegsüberreaktion in allergischen Schafen.

Der VLA-4 abhängige Zelladhäsionsmechanismus wurde ebenfalls in einem Primatenmodell der inflammatory bowel disease (IBD) untersucht. In diesem Modell, das der ulcerativen Colitis im Menschen entspricht, ergab die Gabe von anti-VLA-4-Antikörpern eine signifikante Reduktion der akuten Entzündung.

Darüber hinaus konnte gezeigt werden, daß die VLA-4-abhängige Zelladhäsion bei den folgenden klinischen Konditionen einschließlich der folgenden chronischen entzündlichen Prozesse eine Rolle spielt: Rheumatoide Arthritis (Cronstein und Weismann, Arthritis Rheum. 1993, 36, 147; Elices et al., J. Clin. Invest. 1994, 93, 405), Diabetes mellitus (Yang et al., Proc. Natl. Acad. Sci. USA 1993, 90, 10494), systemischer Lupus erythematosus (Takeuchi et al., J. Clin. Invest. 1993, 92, 3008), Allergien vom verzögerten Typ (Typ IV-Allergie) (Elices et al., Clin. Exp. Rheumatol. 1993, 11, S77), multiple Sklerose (Yednock et al., Nature 1992, 356, 63), Malaria (Ockenhouse et al., J. Exp. Med. 1992, 176,1183), Arteriosklerose (O'Brien et al., J. Clin. Invest. 1993, 92, 945), Transplantation (Isobe et al., Transplantation Proceedings 1994, 26, 867-868), verschiedene Malignitäten, zum Beispiel Melanom (Renkonen et al., Am. J. Pathol. 1992, 140, 763), Lymphom (Freedman et al., Blood 1992, 79, 206) und andere (Albelda et al., J. Cell Biol. 1991, 114, 1059).

Eine VLA-4-Blockierung durch geeignete Antagonisten bietet danach effektive therapeutische Möglichkeiten, insbesondere zum Beispiel verschiedene entzündliche Konditionen einschließlich Asthma und IBD zu behandeln. Die besondere Relevanz von VLA-4-Antagonisten für die Behandlung der rheumatoiden Arthritis ergibt sich dabei, wie bereits gesagt, aus der Tatsache, daß Leukozyten aus dem Blut zunächst an endotheliale Zellen anheften müssen, ehe sie in das Synovium auswandern können, und daß bei dieser Anheftung der VLA-4-Rezeptor eine Rolle spielt. Darauf, daß durch Entzündungsagenzien auf endothelialen Zellen VCAM-1 induziert wird (Osbom, Cell 1990, 62, 3; Stoolman, Cell 1989, 56, 907), und auf die Rekrutierung verschiedener Leukozyten in Infektionsgebiete und Entzündungsherde wurde bereits oben eingegangen. T-Zellen adherieren dabei an aktiviertes Endothel hauptsächlich über die LFA-1/ICAM-1- und VLA-4IVCAM-1-Adhäsionsmechanismen (Springer, Cell 1994, 76, 301). Auf den meisten synovialen T-Zellen ist die Bindungskapazität von VLA-4 für VCAM-1 bei der rheumatoiden Arthritis erhöht (Postigo et al., J. Clin. Invest. 1992, 89, 1445). Zusätzlich wurde eine verstärkte Anheftung von synovialen T-Zellen an Fibronektin beobachtet (Laffon et al., J. Clin. Invest. 1991, 88, 546; Morales-Ducret et al., J. Immunol. 1992, 149, 1424). VLA-4 ist also hochreguliert sowohl im Rahmen seiner Expression als auch hinsichtlich seiner Funktion auf T-Lymphozyten der rheumatoiden Synovialmembran. Die Blockierung der Bindung von VLA-4 an seine physiologischen Liganden VCAM-1 und Fibronektin ermöglicht eine effektive Verhinderung oder Linderung von artikulären Entzündungsprozessen. Dies wird auch durch Experimente mit dem Antikörper HP2/1 an Lewis-Ratten mit Adjuvanz-Arthritis bestätigt, bei denen eine effektive Krankheitsprävention beobachtet wurde (Barbadillo et al., Springer Semin. Immunopathol. 1995, 16, 427). VLA-4 stellt also ein wichtiges therapeutisches Zielmolekül dar.

Die oben erwähnten VLA-4-Antikörper und der Einsatz von Antikörpern als VLA-4-Antagonisten sind in den Patentanmeldungen WO-A-93/13798, WO-A-93115764, WO-A-94/16094, WO-A-94/17828 und WO-A-95/19790 beschrieben. In den Patentanmeldungen WO-A-94/15958, WO-A-95/15973, WO A-96/00581, WO-A-96/06108 und WO-A-96/20216 werden peptidische Verbindungen als VLA-4-Antagonisten beschrieben. Der Einsatz von Antikörpern und peptidischen Verbindungen als Arzneimitteln ist aber mit Nachteilen behaftet, zum Beispiel mangelnder oraler Verfügbarkeit, leichter Abbaubarkeit oder immunoger Wirkung bei längerfristiger Anwendung. Es besteht somit Bedarf nach VLA-4-Antagonisten mit einem günstigen Eigenschaftsprofil für einen Einsatz in der Therapie und Prophylaxe.

In der WO-A-94/21607 und der WO-A-95/14008 sind substituierte 5-Ring-Heterocyclen beschrieben, in der EP-A-449 079, der EP-A-530 505 (US-A-5 389 614), der WO-A-93/18057, der EP-A-566 919 (US-A-5 397 796), der EP-A-5580 008 (US-A-5 424 293) und der EP-A-584 694 (US-A-5 554 594) sind Hydantoinderivate beschrieben, die thrombozytenaggregationshemmende Wirkungen aufweisen. In der EP-A-842 943 (deutsche Patentanmeldung 19647380.2) wird beschrieben, daß derartige Verbindungen überraschenderweise auch die Leukozytenadhäsion hemmen und VLA-4-Antagonisten sind. Verbindungen, die einen Hydantoinring oder einen anderen 5-Ring-Heterocyclus enthalten und VLA-4-Antagonisten sind und die Leukozytenadhäsion hemmen, werden auch in der EP-A-842 944 und der EP-A-842 945 beschrieben. Weitere Untersuchungen zeigten, daß auch die Verbindungen der vorliegenden Erfindung starke Hemmstoffe der Leukozytenadhäsion und VLA-4-Antagonisten sind.

Die vorliegende Erfindung betrifft Verbindungen der Formel I, 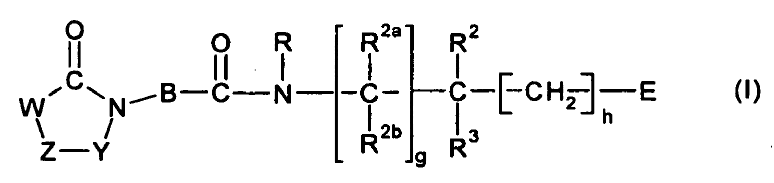 in der
W für R¹-A-C(R¹³) und darin A für den zweiwertigen Phenylenrest steht;
Y für eine Carbonylgruppe steht;
Z für N(R⁰) steht;
B für einen zweiwertigen Methylenrest steht, der unsubstituiert ist oder durch einen (C₁-C₆)-Alkylrest substituiert ist;
E für R¹⁰CO steht;
R und R^{b} unabhängig voneinander für Wasserstoff oder (C₁-C₄)-Alkyl stehen;
R⁰ für im Arylrest gegebenenfalls substituiertes (C₆-C₁₂)-Aryl-(C₁-C₂)-alkyl steht;
R¹ für H,N-C(=NH), H₂N-C(=N-OH), CH₃O-CO-NH-C(=NH), H₂N-C(=NH)-NH oder H₂N-CH₂ steht;
R², R^{2a} und R^{2b} für Wasserstoff stehen;
R³ für R¹¹NH oder CO-R⁵-Het steht;
R⁵ für den zweiwertigen Rest einer natürlichen oder unnatürlichen α-Aminosäure mit einer lipophilen Seitenkette steht, wobei freie funktionelle Gruppen durch in der Peptidchemie übliche Schutzgruppen geschützt sein können oder als Ester oder Amide vorliegen können, und wobei das Stickstoffatom der N-terminalen Aminogruppe einen Rest R^{b} trägt;
R¹⁰ für Hydroxy, (C₁-C₄)-Alkoxy, Phenoxy, Benzyloxy oder (C₁-C₄)-Alkylcarbonyloxy-(C₁-C₄)-alkoxy steht;
R¹¹ für R¹⁵O-CO oder R¹⁵S(O)₂ steht;
R¹³ für (C₁-C₆)-Alkyl steht;
R¹⁵ für R¹⁶-(C₁-C₃)-alkyl oder für R¹⁶ steht;
R¹⁶ für den Rest eines 5-gliedrigen bis 6-gliedrigen monocyclischen Ringes oder für den Rest eines 7-gliedrigen bis 12-gliedrigen bicyclischen oder tricyclischen Ringes steht, wobei diese Ringe gesättigt sind und auch durch einen oder zwei gleiche oder verschiedene (C₁-C₄)-Alkylreste substituiert sein können;
Het für den Rest eines über ein Ring-Stickstoffatom gebundenen 5-gliedrigen oder 6-gliedrigen monocyclischen Heterocyclus steht, der gesättigt ist und der gegebenenfalls ein zusätzliches Ring-Heteratom aus der Reihe Sauerstoff und Schwefel enthalten kann und der durch einen oder zwei gleiche oder verschiedene (C₁-C₄)-Alkylreste substituiert sein kann;
g und h unabhängig voneinander für 0 oder 1 stehen;
in allen ihren stereoisomeren Formen und Mischungen davon in allen Verhältnissen, und ihre physiologisch verträglichen Salze.

Alkylreste können geradkettig oder verzweigt sein. Dies gilt auch, wenn sie Substituenten tragen oder als Substituenten anderer Reste auftreten, beispielsweise in Alkoxyresten, Alkoxycarbonylresten oder Arylalkylresten. Beispiele für geeignete (C₁-C,₈)-Alkylreste sind: Methyl, Ethyl, n-Propyl, n-Butyl, n-Pentyl, n-Hexyl, Heptyl, Octyl, Decyl, Undecyl, Dodecyl, Tridecyl, Pentadecyl, Hexadecyl, Heptadecyl, Octadecyl, Isopropyl, Isobutyl, Isopentyl, Isohexyl, 3-Methylpentyl, 2,3,5-Trimethylhexyl, sec-Butyl, tert-Butyl, tert-Pentyl, Neopentyl. Bevorzugte Alkylreste sind Methyl, Ethyl, n-Propyl, Isopropyl, n-Butyl, Isobutyl, sec-Butyl, tert-Butyt. Beispiele für durch einen Alkylrest substituiertes Methylen sind zum Beispiel Methylen, das durch eine Methylgruppe (= Methyl-methylen oder 1,1-Ethylen oder Ethyliden) substituiert ist, und Methylen, das durch eine Ethylgruppe, eine Isopropylgruppe, eine Isobutylgruppe oder eine tert-Butylgruppe substituiert ist.

Beispiele für Cycloalkylreste sind insbesondere Cyclopentyl und Cyclohexyl, die auch beispielsweise durch (C₁-C₄)-Alkyl substituiert sein können. Als Beispiele für substituierte Cycloalkylreste seien 4-Methylcyclohexyl und 2,3-Dimethylcyclopentyl genannt.

Steht R¹⁶ für den Rest eines gesättigten monocyclischen Ringes, so handelt es sich um einen Cycloalkylrest. Die Ringe können 5 oder 6 Ringglieder enthalten. Die für R¹⁶ stehenden Reste von 7-gliedrigen bis 12-gliedrigen bicyclischen und tricyclischer Ringen werden formal durch Abstraktion eines Wasserstoffatoms aus Bicyclen bzw. Tricyclen erhalten. Die zugrunde liegenden Bicyclen und Tricyclen enthalten als Ringglieder nur Kohlenstoffatome und sind Bicycloalkane oder Tricycloalkane. Die Bicycloalkane und Tricycloalkane können unsubstituiert sein oder in beliebigen geeigneten Positionen durch eine oder zwei gleiche oder verschiedene (C₁-C₄)-Alkylgruppen, zum Beispiel Methylgruppen oder lsopropylgruppen, bevorzugt Methylgruppen, substituiert sein. Die freie Bindung des bicyclischen oder tricyclischen Restes kann sich in einer beliebigen Position des Moleküls befinden, der Rest kann also über ein Brückenkopfatom oder ein Atom in einer Brücke gebunden sein. Die freie Bindung kann sich auch in einer beliebigen stereochemischen Position befinden, beispielsweise in einer exo-Position oder einer endo-Position. Entsprechendes gilt wiederum für die monocyclischen Ringe.

Beispiele für Grundkörper bicyclischer Ringsysteme, von denen sich ein für R¹⁶ stehender bicyclischer Rest ableiten kann, sind das Norbornan (= Bicyclo[2.2.1]heptan), das Bicyclo[2.2.2]octan und das Bicyclo[3.2.1]octan.

Beispiele für Systeme, von denen sich ein für R¹⁶ stehender tricyclischer Rest ableiten kann, sind das Twistan (= Tricyclo[4.4.0.0^{3,8}]decan), das Adamantan (= Tricyclo[3.3.1.1^{3,7}]decan), das Noradamantan (= Tricyclo[3.3.1.0^{3,7}]nonan), das Tricyclo[2.2.10^{2,6}]heptan, das Tricyclo[5.3.2.0^{4,9}]dodecan und das Tricyclo[5.4.0.0^{2,9}]undecan.

Bevorzugt leiten sich für R¹⁶ stehende bicyclische oder tricyclische Reste von verbrückten Bicyclen bzw. Tricyclen ab, also von Systemen, in denen Ringe zwei oder mehr als zwei Atome gemeinsam haben. Im einzelnen bevorzugte Reste sind der 2-Norbornylrest, sowohl derjenige mit der freien Bindung in der exo-Position als auch derjenige mit der freien Bindung in der endo-Position, der 2-Bicyclo[3.2.1]octylrest, der 1-Adamantylrest, der 2-Adamantylrest, der Noradamantylrest, zum Beispiel der 3-Noradamantylrest, und der Homoadamantylrest. Darüber hinaus bevorzugt sind der 1- und der 2-Adamantylrest.

(C₆-C₁₄)-Arylgruppen sind beispielsweise Phenyl, 1-Naphthyl, 2-Naphthyl, Biphenylyl, Anthryl oder Fluorenyl, (C₆-C₁₂)-Arylgruppen sind beispielsweise Phenyl, Naphthyl oder Biphenylyl, (C₆-C₁₀)-Arylgruppen sind beispielsweise Phenyl oder Naphthyl. Bevorzugte Arylreste sind insbesondere 2-Biphenylyl, 3-Biphenylyl, 4-Biphenylyl, 1-Naphthyl, 2-Naphthyl und insbesondere Phenyl. Arylreste, insbesondere Phenylreste, können einfach oder mehrfach, bevorzugt einfach, zweifach oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Methylendioxy, Ethylendioxy, Cyan, Hydroxycarbonyl, Aminocarbonyl, (C₁-C₄)-Alkoxycarbonyl, Phenyl, Phenoxy, Benzyl, Benzyloxy, (R^{d}O)₂P(O), (R^{d}O)₂P(O)-O-, Tetrazolyl substituiert sein, wobei R^{d} wie R und R^{b} definiert ist. Entsprechendes gilt beispielsweise für Reste wie Arylalkyl. Arylalkylreste sind insbesondere Benzyl sowie 1- und 2-Naphthylmethyl, 2-, 3- und 4-Biphenylylmethyl, ferner 1-Phenylethyl und 2-Phenylethyl, die alle auch substituiert sein können.

Substituierte Arylalkylreste sind beispielsweise durch einen oder mehrere (C₁-C₈)-Alkylreste, insbesondere (C₁-C₄)-Alkylreste, im Arylteil substituiertes Benzyl und Naphthylmethyl, zum Beispiel 2-, 3- und 4-Methylbenzyl, 4-lsobutylbenzyl, 4-tert-Butylbenzyl, 4-Octylbenzyl, 3,5-Dimethylbenzyl, Pentamethylbenzyl, 2-, 3-, 4-, 5-, 6-, 7- und 8-Methyl-1-naphthylmethyl, 1-, 3-, 4-, 5-, 6-, 7- und 8-Methyl-2-naphthylmethyl, durch einen oder mehrere (C₁-C₈)-Alkoxyreste, insbesondere (C₁-C₄)-Alkoxyreste, im Arylteil substituiertes Benzyl und Naphthylmethyl, zum Beispiel 4-Methoxybenzyl, 4-Neopentyloxybenzyl, 3,5-Dimethoxybenzyl, 3,4-Methylendioxybenzyl, 2,3,4-Trimethoxybenzyl, 2-, 3- und 4-Nitrobenzyl, Halobenzyl, zum Beispiel 2-, 3- und 4-Chlorbenzyl und 2-, 3- und 4-Fluorbenzyl, 3,4-Dichlorbenzyl, Pentafluorbenzyl, Trifluorrnethylbenzyl, zum Beispiel 3- und 4-Trifluormethylbenzyl oder 3,5-Bis(trifluormethyl)benzyl. Substituierte Arylalkylreste können aber auch unterschiedliche Substituenten aufweisen. Die in den beispielhaft genannten substituierten Arylalkylresten enthaltenen substituierten Arylreste stellen zugleich Beispiele für substituierte Arylreste dar.

In monosubstituierten Phenylresten kann sich der Substituent in der 2-Position, der 3-Position oder der 4-Position befinden, wobei die 3-Position und die 4-Position bevorzugt sind. Ist Phenyl zweifach substituiert, können sich die Substituenten in 2,3-Position, 2,4-Position, 2,5-Position, 2,6-Position, 3,4-Position oder 3,5-Position befinden. Bevorzugt sind in zweifach substituierten Phenylresten die beiden Substituenten in 3,4-Position angeordnet. Ist Phenyl dreifach substituiert, so können sich die Substituenten beispielsweise in 2,3,4-Position, 2,3,5-Position, 2,3,6-Position, 2,4,5-Position, 2,4,6-Position oder 3,4,5-Position befinden. Entsprechendes gilt für substituierte Phenylenreste. Phenylenreste können zum Beispiel als 1,4-Phenylen oder als 1,3-Phenylen vorliegen.

Für den Rest Het stehende heterocylische Reste können an Kohlenstoffatomen unsubstituiert oder einfach oder zweifach durch gleiche oder verschiedene (C₁-C₄)-Alkyl-Substituenten substituiert sein. Schwefelatome können zum Sulfoxid oder zum Sulfon oxidiert sein. Beispiele für den Rest Het sind 1-Pyrrolidinyl, 1-Piperidinyl, 4-Morpholinyl, 4-Thiomorpholinyl, 1-Oxo-4-thiomorpholinyl, 1,1-Dioxo-4-thiomorpholinyl, 2,6-Dimethyl-l-piperidinyl, 3,3-Dimethyl-4-morpholinyl.

Halogen steht für Fluor, Chlor, Brom oder Iod, insbesondere für Fluor oder Chlor.

Der zweiwertige Rest einer α-Aminosäure, der für R⁵ steht, wird wie in der Peptidchemie üblich aus der entsprechenden Aminosäure erhalten, indem von deren N-terminalen Aminogruppe formal ein Wasserstoffatom entfernt wird und von oder C-terminalen Carbonsäuregruppe die Hydroxygruppe entfernt wird. Über die so entstehende freie Bindung an der Aminogruppe ist diese Gruppe dann peptidartig durch eine Amidbindung mit der Carbonylgruppe der Nachbargruppe verknüpft, wobei die in R⁵ enthaltene N-terminale Aminogruppe an die CO-Gruppe in der Gruppe CO-R⁵-Het gebunden ist. Entsprechend ist die aus der Carbonsäuregruppe durch das Entfernen der Hydroxygruppe formal entstehende Carbonylgruppe über ihre freie Bindung peptidartig durch eine Amidbindung mit einem Stickstoffatom der Nachbargruppe Het verknüpft. Wie oben angegeben tragen die Stickstoffatome der Amidbindungen, also der Gruppen CO-N(R^{b}) , durch die die CO-Gruppe in der Gruppe CO-R⁵-Het mit der Gruppe R⁵ verknüpft ist, den Substituenten R^{b}, der für Wasserstoff oder (C₁-C₄)-Alkyl, zum Beispiel Methyl, stehen kann.

Die natürlichen und unnatürlichen Aminosäuren können in allen stereochemischen Formen vorliegen, beispielsweise in der D-Form, der L-Form oder in Form einer Mischung von Stereoisomeren, zum Beispiel in Form eines Racemats. Als in Betracht kommende Aminosäuren, von denen sich R⁵ ableiten kann, seien beispielsweise genannt (vgl. Houben-Weyl, Methoden der organischen Chemie, Band 15/1 und 15/2, Georg Thieme Verlag, Stuttgart, 1974):
Aad, Abu, Ach, Acp, Aib, Ala, ΔAla, Alg, All, Ama, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, hAla, hArg.. hCys, hGln, hGlu, His, hIle, hLeu, hLys, hMet, hPhe, hPro, hSer, hThr, hTrp, hTyr, Hyl, Hyp, 3Hyp, Ile, Iva, Lant, Lcn, Leu, Lys, ΔLys, Met, Min, nArg, Nle, Nva, Oly, Orn, Pan, Pec, Pen, Phe, Phg, Pic, Pro, Pro, Pse, Pya, Pyr, Pza, Sar, Sec, Sem, Ser, Thi, Thr, Thy, Thx, Tia, Tle, Tly, Trp, Trta, Tyr, Val, tert-Butylglycin (Tbg), Neopentylglycin (Npg), Cyclohexylglycin (Chg), Cyclohexylalanin (Cha), 2-Thienylalanin (Thia), 2,2-Diphenylaminoessigsäure, 2-(p-Tolyl)-2-phenylaminoessigsäure, 2-(p-Chlorphenyl)-aminoessigsäure.

Steht R⁵ für den Rest einer natürlichen oder unnatürlichen α-Aminosäure, die am α-Kohlenstoffatom noch ein Wasserstoffatom trägt, dann liegt der zweiwertige Rest -N(R^{b})-CH(SC)-CO- vor, in dem SC für die Seitenkette der α-Aminosäure steht, also zum Beispiel für einen der Substituenten, die in der α-Position der vorstehend genannten, in der α-Position unverzweigten α-Aminosäuren enthalten sind. Beispiele für Seitenketten sind Alkylreste, zum Beispiel die Methylgruppe im Alanin oder die Isopropylgrupppe im Valin, der Benzylrest im Phenylalanin, der Phenylrest im Phenylglycin, der 4-Aminobutylrest im Lysin oder die Hydroxycarbonylmethylgruppe in der Asparaginsäure. Solche Seitenketten und damit die Aminosäuren können im Sinne der vorliegenden Erfindung außer durch ihre chemische Struktur zum Beispiel auch aufgrund ihrer physikochemischen Eigenschaften zu einer Gruppe zusammengefaßt werden, beispielsweise können lipophile Seitenketten von hydrophilen Seitenketten, die polare Gruppen enthalten, unterschieden werden. Beispiele für lipophile Seitenketten, die in für R⁵ stehenden Aminosäuren enthalten sein können, sind Alkylreste, Arylalkylreste oder Arylreste, zum Beispiel (C₁-C₆)-Alkylreste, im Arylrest gegebenenfalls substituierte (C₆-C₁₂)-Aryl-(C₁-C₄)-alkylreste und gegebenenfalls substituierte (C₆-C₁₂)-Arylreste, wobei für diese Reste die obigen Erläuterungen gelten.

Säuregruppen in natürlichen oder unnatürlichen Aminosäuren können auch als Ester oder Amide vorliegen, zum Beispiel als (C₁-C₄)-Alkylester wie zum Beispiel als Methylester, Ethylester, Isopropylester, Isobutylester oder tert-Butylester, Benzylester, unsubstituierte Amide, Methylamide, Ethylamide, Semicarbazide oder ω-Amino-(C₂-C₈)-alkylamide.

Funktionelle Gruppen der Aminosäuren können geschützt vorliegen. Geeignete Schutzgruppen wie zum Beispiel Urethanschutzgruppen, Carboxylschutzgruppen und Seitenkettenschutzgruppen sind bei Hubbuch, Kontakte (Merck) 1979, Nr. 3, Seiten 14 bis 23, und bei Büllesbach, Kontakte (Merck) 1980, Nr. 1, Seiten 23 bis 35, beschrieben. Insbesondere seien genannt: Aloc, Pyoc, Fmoc, Tcboc, Z, Boc, Ddz, Bpoc, Adoc, Msc, Moc, Z(NO₂), Z(Halₙ), Bobz, lboc, Adpoc, Mboc, Acm, tert-Butyl, OBzl, ONbzl, OMbzl, Bzl, Mob, Pic, Trt.

Die Verbindungen der Formel I können in stereoisomeren Formen vorliegen. Enthalten die Verbindungen der Formel ein oder mehrere Asymmetriezentren, so können diese unabhängig voneinander die S-Konfiguration oder die R-Konfiguration aufweisen. Zur Erfindung gehören alle möglichen Stereoisomeren, zum Beispiel Enantiomere und Diastereomere, und Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Gegebenenfalls kann vor einer Trennung von Stereoisomeren eine Derivatisierung unter Verwendung eines chiralen Reagenzes erfolgen und die erhaltenen diastereomeren Verbindungen können dann nach üblichen Methoden, zum Beispiel durch Kristallisation oder Chromatographie, getrennt werden. Die Trennung eines Stereoisomerengemisches kann auf der Stufe der Verbindungen der Formel I erfolgen oder auf der Stufe eines Zwischenprodukts im Verlaufe der Synthese.

Bei Vorliegen von beweglichen Wasserstoffatomen umfaßt die vorliegende Erfindung auch alle tautomeren Formen der Verbindungen der Formel I, zum Beispiel Lactam/Lactim-Tautomere.

Enthalten die Verbindungen der Formel I eine oder mehrere saure oder basische Gruppen, so sind auch die entsprechenden physiologisch oder toxikologisch verträglichen Salze Gegenstand der Erfindung, insbesondere die pharmazeutisch verwendbaren Salze. So können die Verbindungen der Formel I, die eine oder mehrere saure Gruppen enthalten, zum Beispiel Carbonsäuregruppen oder Sulfonsäuregruppen, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze oder Salze mit Ammoniak oder organischen Aminen wie beispielsweise Ethylamin, Ethanolamin, Triethanolamin oder Aminosäuren.

Verbindungen der Formel I, die eine oder mehrere basische, das heißt protonierbare, Gruppen enthalten, zum Beispiel Aminogruppen, Amidinogruppen oder Guanidinogruppen, können in Form ihrer Säureadditionssalze mit anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bernsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw. Enthalten die Verbindungen der Formel I gleichzeitig saure und basische Gruppen im Molekül, so gehören neben den geschilderten Salzformen auch innere Salze oder Betaine zu der Erfindung.

Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen.

Die vorliegende Erfindung umfaßt weiterhin alle Solvate von Verbindungen der Formel I, zum Beispiel Hydrate oder Addukte mit Alkoholen, sowie Derivate der Verbindungen der Formel I, zum Beispiel Ester, Pro-Drugs und aktive Metabolite.

Die einzelnen Strukturelemente in der Formel haben bevorzugt die folgenden Bedeutungen.

B steht bevorzugt für einen zweiwertigen Methylenrest, der substituiert ist. Ist ein für B stehender zweiwertiger Methylenrest durch einen (C₁-C₆)-Alkylrest substituiert, so ist dieser Alkylrest geradkettig oder verzweigt und enthält 1, 2, 3, 4, 5 oder 6 Kohlenstoffatome.

R und R^{b} stehen bevorzugt unabhängig voneinander für Wasserstoff, Methyl oder Ethyl.

R¹³ steht bevorzugt für den Methylrest.

R¹⁵ steht bevorzugt für R¹⁶-(C₁)-alkyl oder für R¹⁶.

Wenn R³ für R¹¹NH steht, steht bevorzugt g für 1 und h für 0. Wenn R³ für CO-R⁵-Het steht, steht bevorzugt g für 0 und h für 1.

Von den Verbindungen der Formel I sind solche Verbindungen bevorzugt, in denen einer oder mehrere der Reste bevorzugte Bedeutungen haben, wobei alle Kombinationen von bevorzugten Substituentenbedeutungen Gegenstand der vorliegenden Erfindung sind. Besonders bevorzugte Verbindungen der Formel I sind insbesondere solche, worin gleichzeitig
W für R¹-A-C(R¹³) und darin A für den zweiwertigen Phenylenrest steht;
Y für eine Carbonylgruppe steht;
Z für N(R⁰) steht;
B für einen zweiwertigen Methylenrest steht, der unsubstituiert ist oder durch einen (C₁-C₆)-Alkyl-Rest substituiert ist;
E für R¹⁰CO steht;
R und R^{b} unabhängig voneinander für Wasserstoff oder Methyl stehen;
R⁰ für im Arylrest gegebenenfalls substituiertes (C₆-C₁₂)-Aryl-(C₁-C₂)-alkyl steht;
R¹ für H₂N-C(=NH), H₂N-C(=NOH), CH₃O-CO-NH-C(=NH), H₂N-C(=NH)-NH oder H₂N-CH₂ steht;
R², R^{2a} und R^{2b} für Wasserstoff stehen
R³ für R¹¹NH oder CO-R⁵-Het steht;
R⁵ für den zweiwertigen Rest einer natürlichen oder unnatürlichen α-Aminosäure mit einer lipophilen Seitenkette steht, wobei freie funktionelle Gruppen durch in der Peptidchemie übliche Schutzgruppen geschützt sein können oder als Ester oder Amide vorliegen können, und wobei das Stickstoffatom der N-terminalen Aminogruppe einen Rest R^{b} trägt;
R¹⁰ für Hydroxy, (C₁-C₄)-Alkoxy, Phenoxy, Benzyloxy oder (C₁-C₄)-Alkylcarbonyloxy-(C₁-C₄)-alkoxy steht;
R¹¹ für R¹⁵O-CO steht;
R¹³ für (C₁-C₆)-Alkyl steht;
R¹⁵ für R¹⁶ oder R¹⁶-CH₂ steht;
R¹⁶ für Cyclopentyl, Cyclohexyl, 1-Adamantyl, 2-Adamantyl oder Noradamantyl steht; Het für den Rest eines über ein Ring-Stickstoffatom gebundenen 5-gliedrigen oder 6-gliedrigen monocyclischen Heterocyclus steht, der gesättigt ist und der gegebenenfalls ein Sauerstoffatom als zusätzliches Ring-Heteratom enthalten kann und der durch einen oder zwei gleiche oder verschiedene (C₁-C₄)-Alkylreste substituiert sein kann;
in allen ihren stereoisomeren Formen und Mischungen davon in allen Verhältnissen, und ihre physiologisch verträglichen Salze.

Generell sind Verbindungen der Formel I bevorzugt, die an Chiralitätszentren, zum Beispiel an dem die Reste R² und R³ tragenden chiralen Kohlenstoffatom und/oder an dem Zentrum W im 5-Ring-Heterocyclus in der Formel I, eine einheitliche Konfiguration aufweisen.

Die Verbindungen der Formel I können beispielsweise hergestellt werden durch Fragmentkondensation einer Verbindung der Formel II 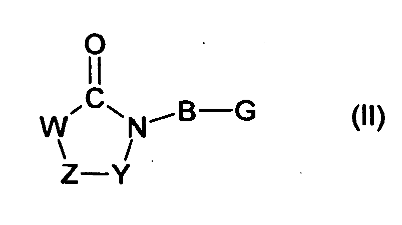 mit einer Verbindung der Formel III, 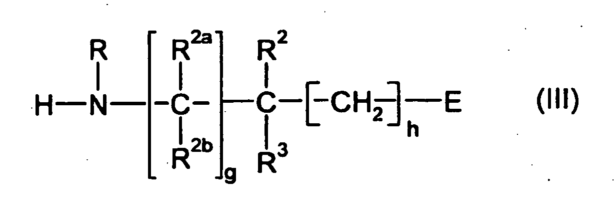 wobei in den Formeln II und III die Gruppen W, Y, Z, B, E, R, R², R^{2a}, R^{2b} und R³ sowie g und h wie oben angegeben definiert sind oder auch funktionelle Gruppen in geschützter Form oder in Form von Vorstufen enthalten sein können, und wobei G für Hydroxycarbonyl, (C₁-C₆)-Alkoxycarbonyl oder aktivierte Carbonsäurederivate wie Säurechloride oder Aktivester steht. In den Verbindungen der Formel III kann, wenn Verbindungen der Formel I hergestellt werden sollen, in denen R³ in der Formel I für CO-R⁵-Het steht, beispielsweise auch der Rest R³ zunächst für eine in geschützter Form vorliegende Hydroxycarbonylgruppe stehen und dann erst nach der Kondensation der Verbindungen der Formeln II und III in einem oder mehreren weiteren Kondensationsschritten die gewünschte Gruppe R³ aufgebaut werden.

Zur Kondensation der Verbindungen der Formel II mit denen der Formel III verwendet man vorteilhafterweise die dem Fachmann an sich wohlbekannten Kupplungsmethoden der Peptidchemie (siehe zum Beispiel Houben-Weyl, Methoden der Organischen Chemie, Band 15/1 und 15/2, Georg Thieme Verlag, Stuttgart, 1974). Als Kondensationsmittel kommen zum Beispiel Carbonyldiimidazol, Carbodiimide wie Dicyclohexylcarbodiimid oder Diisopropylcarbodiimid, das O-((Cyan(ethoxycarbonyl)methylen)amino)-N,N,N',N'-tetramethyluronium-tetrafluoroborat (TOTU) oder Propylphosphonsäureanhydrid (PPA) in Frage. Bei der Kondensation ist es in der Regel nötig, daß vorhandene, nicht reagierende Aminogruppen durch reversible Schutzgruppen geschützt werden. Gleiches gilt für nicht an der Reaktion beteiligte Carboxylgruppen, die während der Kondensation bevorzugt als (C₁-C₆)-Alkylester, zum Beispiel tert-Butylester, oder als Benzylester vorliegen. Ein Aminogruppen-Schutz erübrigt sich, wenn die Aminogruppen noch in Form von Vorstufen, zum Beispiel als Nitrogruppen oder Cyangruppen, vorliegen und erst nach der Kupplung zum Beispiel durch Hydrierung gebildet werden. Nach der Kupplung werden die vorhandenen Schutzgruppen in geeigneter Weise abgespalten. Beispielsweise können NO₂-Gruppen (Guanidinoschutz), Benzyloxycarbonylgruppen und Benzylester abhydriert werden. Die Schutzgruppen vom tert-Butyltyp werden sauer abgespalten, während der 9-Fluorenylmethyloxycarbonylrest durch sekundäre Amine entfernt wird.

Verbindungen der Formel II können beispielsweise hergestellt werden, indem man zunächst Verbindungen der Formel IV 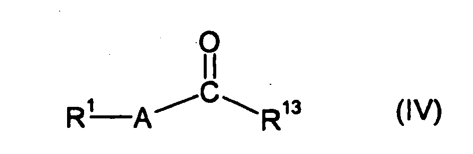 in einer Bucherer-Reaktion zu Verbindungen der Formel V, 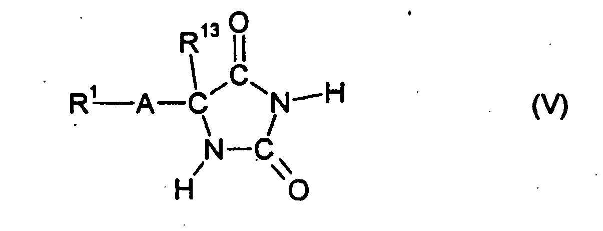 in der ebenso wie in der Formel IV R¹, R¹³ und A wie oben angegeben definiert sind, umsetzt (H. T. Bucherer, V. A. Lieb, J. Prakt: Chem. 141(1934), 5). Verbindungen der Formel VI, 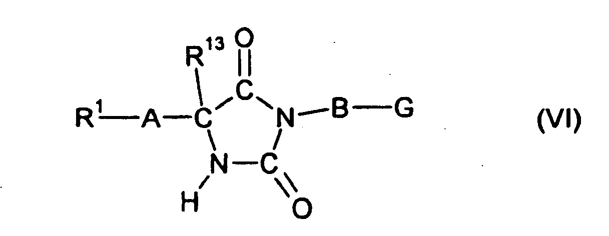 in der R¹, R¹³, A, B und G wie oben angegeben definiert sind, können dann erhalten werden, indem man die Verbindungen der Formel V beispielsweise zunächst mit einem alkylierenden Reagenz umsetzt, das den Rest -B-G in das Molekül einführt. Die Umsetzung von Verbindungen der Formel VI mit einem zweiten Reagenz der Formel R⁰-LG, in der R⁰ die oben angegebenen Bedeutungen hat und LG eine nucleophil substituierbare Abgangsgruppe, zum Beispiel Halogen, insbesondere Chlor oder Brom, (C₁-C₄)-Alkoxy, gegebenenfalls substituiertes Phenoxy oder eine heterocyclische Abgangsgruppe wie zum Beispiel Imidazolyl, darstellt, führt zu den entsprechenden Verbindungen der Formel II. Diese Umsetzungen können analog bekannten, dem Fachmann geläufigen Methoden durchgeführt werden. Je nach dem Einzelfall kann es hierbei, wie bei allen Schritten in der Synthese der Verbindungen der Formel I, angebracht sein, funktionelle Gruppen die zu Nebenreaktionen oder unerwünschten Reaktionen führen könnten, durch eine dem Syntheseproblem angepaßte Schutzgruppenstrategie temporär zu blockieren, was dem Fachmann bekannt ist. Hinsichtlich der Herstellung der Verbindungen der Formeln V und VI in racemischer Form und in enantiomerenreiner Form wird hier insbesondere Bezug genommen auf die entsprechenden Ausführungen in der WO-A-96/33976, die Bestandteil der vorliegenden Offenbarung sind.

Die Aminoverbindungen der Formel III können nach oder analog zu Standardverfahren aus Ausgangsverbindungen aufgebaut werden, die käuflich sind oder nach oder analog zu Literaturvorschriften erhältlich sind.

Verbindungen der Formel I können auch wie folgt erhalten werden:
Durch Reaktion von nach Standardverfahren erhältlichen α-Aminosäuren oder N-substituierten α-Aminosäuren oder bevorzugt deren Ester, zum Beispiel der Methylester, Ethylester, tert-Butylester oder Benzylester, beispielsweise einer Verbindung der Formel VII, 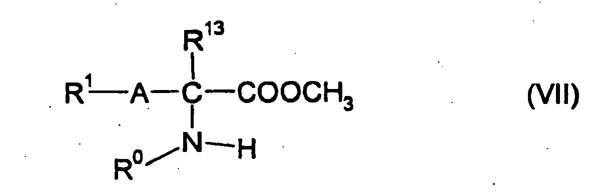 worin R⁰, R¹, R¹³ und A wie oben angegeben definiert sind, mit einem Isocyanat beispielsweise der Formel VIII, 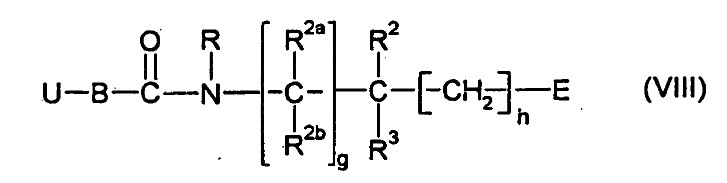 worin B, E, R, R², R^{2a}, R^{2b}, R³, g und h wie oben angegeben definiert sind und U für Isocyanato steht, erhält man Harnstoffderivate, beispielsweise der Formel IX, 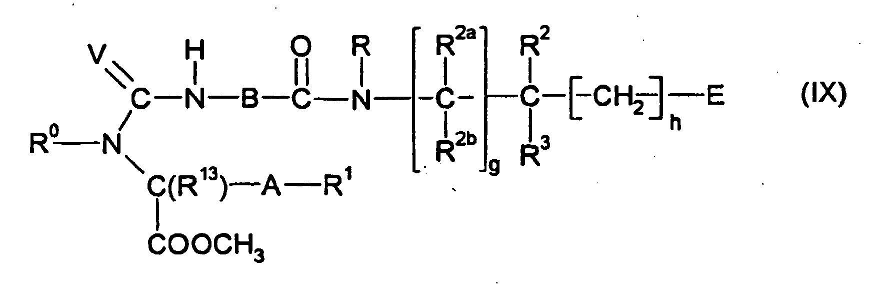 für die die oben angegebenen Definitionen gelten und in der V Sauerstoff Schwefe bedeutet, und die durch Erhitzen mit Säure unter Verseifung der Esterfunktionen zu Verbindungen der Formel la 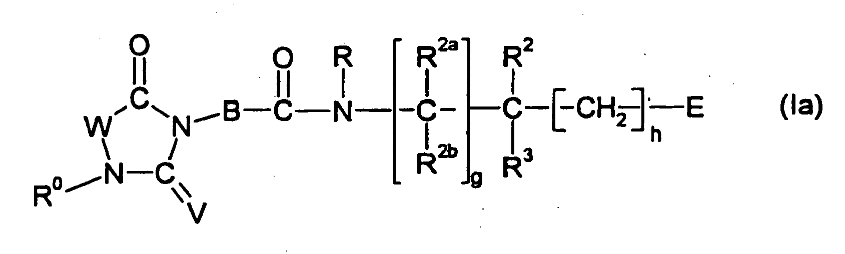 cyclisiert werden, in der V Sauerstoff bedeutet, W für R¹-A-C(R¹³) steht und für die ansonsten die oben angegebenen Bedeutungen gelten. Die Cyclisierung der Verbindungen der Formel IX zu den Verbindungen der Formel la kann auch durch Behandlung mit Basen in inerten Lösungsmitteln durchgeführt werden, zum Beispiel durch Behandlung mit Natriumhydrid in einem aprotischen Lösungsmittel wie Dimethylformamid.

Während der Cyclisierung können Guanidinogruppen durch Schutzgruppen, zum Beispiel NO₂, blockiert werden. Aminogruppen können in geschützer Form oder zum Beispiel noch als NO₂-Funktion oder Cyanfunktion vorliegen, die später zur Aminogruppe reduziert oder im Falle der Cyangruppe auch in die Formamidinogruppe umgewandelt werden kann.

Verbindungen der Formel I können auch erhalten werden; indem man eine Verbindung der Formel VII mit einem Isocyanat oder Isothiocyanat der Formel X 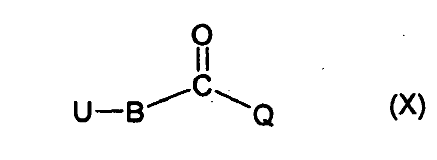 umsetzt, in der B und U wie oben für die Formel VIII angegeben definiert sind und Q eine Alkoxygruppe, zum Beispiel eine (C₁-C₄)-Alkoxygruppe wie Methoxy, Ethoxy oder tert-Butoxy, eine (C₆-C₁₄)-Aryloxygruppe, zum Beispiel Phenoxy, oder eine (C₆-C₁₄)-Aryl-(C₁-C₄)-alkoxygruppe, zum Beispiel Benzyloxy, bedeutet. Dabei wird eine Verbindung der Formel XI 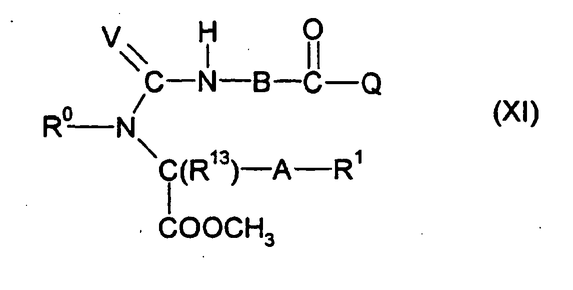 erhalten, in der V, A, B, Q, R⁰, R¹ und R¹³ wie oben für die Formeln IX und X angegeben definiert sind, die dann unter dem Einfluß einer Säure oder einer Base, wie oben für die Cyclisierung der Verbindungen der Formel IX beschrieben, zu einer Verbindung der Formel XII, 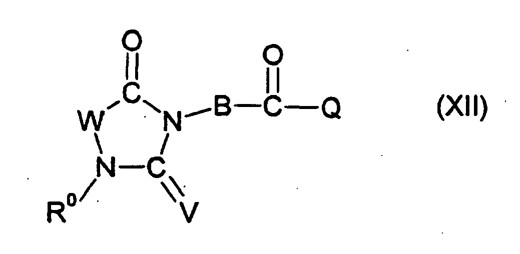 in der W für R¹-A-C(R¹³) steht und V, B, Q und R⁰ wie oben für die Formeln la und X angegeben, definiert sind, cyclisiert wird. Aus der Verbindung der Formel XII wird dann durch Hydrolyse der Gruppe CO-Q zur Carbonsäure COOH und nachfolgende Kupplung mit einer Verbindung der Formel III, wie oben für die Kupplung der Verbindungen der Formeln II und III beschrieben, eine Verbindung der Formel la erhalten. Auch hier können während der Cyclisierung funktionelle Gruppen in geschützter Form vorliegen oder in Form von Vorstufen vorliegen.

Eine weitere Methode zur Herstellung von Verbindungen der Formel Ia ist beispielsweise die Umsetzung von Verbindungen der Formel XIII,

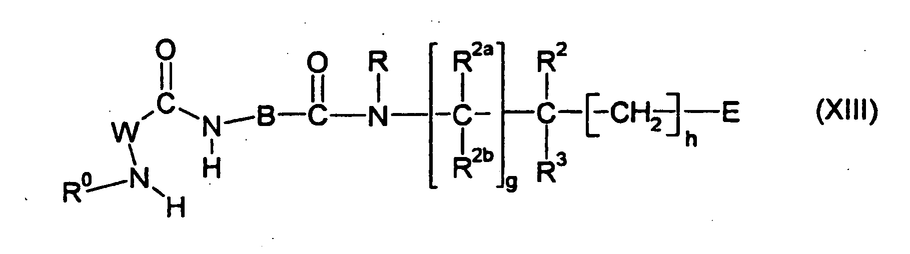

in der W für R¹-A-C(R¹³) steht und für die ansonsten die oben angegebenen Definitionen gelten, mit Phosgen oder entsprechenden Äquivalenten (analog S. Goldschmidt und M. Wick, Liebigs Ann. Chem. 575 (1952), 217-231 und C. Tropp, Chem. Ber. 61 (1928), 1431-1439).

Die Überführung einer Aminofunktion in die Guanidinofunktion kann mit folgenden Reagenzien durchgeführt werden:
1. O-Methylisoharnstoff (S. Weiss und H. Krommer, Chemiker Zeitung 98 (1974), 617-618)
2. S-Methylisothioharnstoff (R.F. Bome, M.L.Forrester und I.W. Waters, J. Med. Chem. 20 (1977), 771-776)
3. Nitro-S-methylisothioharnstoff (L.S. Hafner und R.E. Evans, J. Org. Chem. 24 (1959) 57)
4. Formamidinosulfonsäure (K. Kim, Y.-T. Lin und H.S. Mosher, Tetrah. Lett. 29 (1988), 3183-3186)
5. 3,5-Dimethyl-1-pyrazolyl-formamidinium-nitrat (F.L. Scott, D.G. O'Donovan und J. Reilly, J. Amer. Chem. Soc. 75 (1953), 4053-4054)
6. N,N-Di-tert-butyloxycarbonyl-S-methyl-isothioharnstoff (R. J. Bergeron und J. S. McManis, J. Org. Chem. 52 (1987), 1700-1703)
7. N-Alkoxycarbonyl-, N,N'-Dialkoxycarbonyl-, N-Alkylcarbonyl- und N,N'-dialkylcarbonyl-S-methyl-isothiohamstoff (H. Wollweber, H. Kölling, E. Niemers, A. Widdig, P. Andrews, H.-P. Schulz und H. Thomas, Arzneim. Forsch./Drug Res. 34 (1984), 531-542).

Amidine können aus den entsprechenden Cyanverbindungen durch Anlagerung von Alkoholen (zum Beispiel Methanol oder Ethanol) in einem sauren wasserfreien Medium (zum Beispiel Dioxan, Methanol oder Ethanol) und anschließende Aminolyse, zum Beispiel Behandlung mit Ammoniak in Alkoholen wie zum Beispiel Isopropanol, Methanol oder Ethanol hergestellt werden (G. Wagner, P. Richter und Ch. Garbe, Pharmazie 29 (1974), 12-55). Eine weitere Methode, Amidine herzustellen, ist die Anlagerung von H₂S an die Cyangruppe, gefolgt von einer Methylierung des entstandenen Thioamids und anschließender Umsetzung mit Ammoniak (DDR-Patent Nr. 235 866). Weiterhin kann Hydroxylamin an die Cyangruppe angelagert werden, wobei N-Hydroxyamidine entstehen, die dann gewünschtenfalls auch, zum Beispiel durch Hydrierung, in die Amidine überführt werden können.

Hinsichtlich der Herstellung der Verbindungen der Formel I wird weiterhin vollinhaltlich Bezug genommen auf die WO-A-96/33 976 und folgende Schriften, in denen Verbindungen mit thrombozytenaggregationshemmender Wirkung beschrieben werden: WO-A-94/21607, WO-A-95/14008, EP-A-449 079, EP-A-530 505 (US-A-5 389 614), WO-A-93/18057. EP-A-566 919 (US-A-5 397 796), EP-A-580 008 (US-A-5 424 293) und EP-A-584 694 (US-A-5 554 594).

Die Verbindungen der Formel I sind wertvolle Arzneimittelwirkstoffe, die sich beispielsweise für die Therapie und Prophylaxe von Entzündungserkrankungen, allergischen Erkrankungen oder Asthma eignen. Die Verbindungen der Formel I und ihre physiologisch verträglichen Salze können erfindungsgemäß am Tier, bevorzugt am Säugetier, und insbesondere am Menschen als Arzneimittel zur Therapie oder Prophylaxe verabreicht werden. Sie können für sich allein, in Mischungen untereinander oder in Form von pharmazeutischen Präparaten verabreicht werden, die eine enterale oder parenterale Anwendung gestatten und die als aktiven Bestandteil eine wirksame Dosis mindestens einer Verbindung der Formel I und/oder ihrer physiologisch verträglichen Salze neben üblichen pharmazeutisch einwandfreien Trägerstoffen und/oder Zusatzstoffen enthalten.

Gegenstand der vorliegenden Erfindung sind daher auch die Verbindungen der Formel I und/oder ihre physiologisch verträglichen Salze zur Verwendung als Arzneimittel, die Verwendung der Verbindungen der Formel I und/oder ihrer physiologisch verträglichen Salze zur Herstellung von Arzneimitteln für die Therapie und Prophylaxe der oben oder im folgenden bezeichneten Krankheiten, zum Beispiel für die Therapie und Prophylaxe von Entzündungserkrankungen, sowie die Verwendung der Verbindungen der Formel I und/oder ihrer physiologisch verträglichen Salze bei der Therapie und Prophylaxe dieser Krankheiten. Weiterhin sind Gegenstand der vorliegenden Erfindung pharmazeutische Präparate, die eine wirksame Dosis mindestens einer Verbindung der Formel I und/oder ihrer physiologisch verträglichen Salze neben üblichen pharmazeutisch einwandfreien Trägerstoffen und/oder Zusatzstoffen enthalten.

Die Arzneimittel können oral, zum Beispiel in Form von Pillen, Tabletten, Filmtabletten, Dragees, Granulaten, Hart- und Weichgelatinekapseln, Lösungen, Sirupen, Emulsionen oder Suspensionen verabreicht werden. Die Verabreichung kann aber auch rektal, zum Beispiel in Form von Suppositorien, oder parenteral, zum Beispiel in Form von Injektions- oder Infusionslösungen, Mikrokapseln oder Rods, oder perkutan, zum Beispiel in Form von Salben, Lösungen oder Tinkturen, oder auf anderem Wege, zum Beispiel in Form von Nasalsprays oder Aerosolmischungen, erfolgen.

Die Herstellung der erfindungsgemäßen pharmazeutischen Präparate erfolgt in an sich bekannter Weise, wobei neben der oder den Verbindungen der Formel I und/oder ihren physiologisch verträglichen Salzen pharmazeutisch inerte anorganische oder organische Trägerstoffe verwendet werden. Für die Herstellung von Pillen, Tabletten, Dragees und Hartgelatinekapseln kann man zum Beispiel Lactose, Maisstärke oder Derivate davon, Talk, Stearinsäure oder deren Salze etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich zum Beispiel Wasser, Alkohole, Glycerin, Polyole, Saccharose, Invertzucker, Glukose, pflanzliche Öle etc. Als Trägerstoffe für Mikrokapseln, Implantate oder Rods eignen sich zum Beispiel Mischpolymerisate aus Glykolsäure und Milchsäure. Die pharmazeutischen Präparate enthalten normalerweise etwa 0,5 bis 90 Gew.-% der Verbindungen der Formel 1 und/oder ihrer physiologisch verträglichen Salze.

Die pharmazeutischen Präparate können neben den Wirkstoffen und Trägerstoffen noch Zusatzstoffe, wie zum Beispiel Füllstoffe, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I und/oder deren physiologisch verträgliche Salze enthalten. Ferner können sie neben mindestens einer Verbindung der Formel 1 und/oder ihren physiologisch verträglichen Salzen noch einen oder mehrere andere therapeutisch oder prophylaktisch wirksame Stoffe enthalten, zum Beispiel Stoffe mit entzündungshemmender Wirkung. Die pharmazeutische Präparate enthalten normalerweise 0,2 bis 500 mg, vorzugsweise 1 bis 100 mg Wirkstoff der Formel I und/oder dessen physiologisch verträgliche Salze.

Die Verbindungen der Formel I haben die Fähigkeit, Zell-Zell- und Zell-Matrix-Interaktionsprozesse zu inhibieren, bei denen Wechselwirkungen zwischen VLA-4 mit seinen Liganden eine Rolle spielen. Die Wirksamkeit der Verbindungen der Formel I kann beispielsweise in einem Assay nachgewiesen werden, indem die Bindung von Zellen, die den VLA-4-Rezeptor aufweisen, zum Beispiel von Leukozyten, an Liganden dieses Rezeptors gemessen wird, zum Beispiel an VCAM-1, das dafür vorteilhafterweise auch gentechnisch hergestellt werden kann. Einzelheiten eines solchen Assay sind weiter unten beschrieben. Insbesondere vermögen die Verbindungen der Formel I die Adhäsion und die Migration von Leukozyten inhibieren, etwa die Anheftung von Leukozyten an endotheliale Zellen, die - wie oben erläutert - über den VCAM-1/VLA-4-Adhäsionsmechanismus gesteuert wird. Außer als Entzündungshemmstoffe eignen sich die Verbindungen der Formel I und ihre physiologisch verträglichen Salze daher generell zur Therapie und Prophylaxe von Krankheiten, die auf der Wechselwirkung zwischen dem VLA-4-Rezeptor und seinen Liganden beruhen oder durch eine Hemmung dieser Wechselwirkung beeinflußt werden können, und insbesondere eignen sie sich für die Therapie und Prophylaxe von Krankheiten, die zumindest teilweise durch ein unerwünschtes Ausmaß an Leukozytenadhäsion und/oder Leukozytenmigration verursacht werden oder damit verbunden sind, oder zu deren Vorbeugung, Linderung oder Heilung die Adhäsion und/oder Migration von Leukozyten verringert werden soll.

Die Verbindungen der Formel I können bei entzündlichen Erscheinungen unterschiedlichster Ursache als Entzündungshemmer eingesetzt werden. Anwendung finden sie beispielsweise zur Therapie oder Prophylaxe der rheumatoiden Arthritis, der inflammatorischen bowel disease (ulcerativen Colitis), des systemischen Lupus erythematosus oder zur Therapie oder Prophylaxe von inflammatorischen Erkrankungen des zentralen Nervensystems wie zum Beispiel der multiplen Sklerose, zur Therapie oder Prophylaxe von Asthma oder von Allergien, zum Beispiel Allergien vom verzögerten Typ (Typ IV-Allergie). Weiterhin eignen sie sich zur Therapie oder Prophylaxe von cardiovaskulären Erkrankungen, der Arteriosklerose, von Restenosen, zur Therapie oder Prophylaxe von Diabetes, zur Verhinderung der Schädigung von Organtransplantaten, zur Hemmung von Tumorwachstum oder Tumormetastasierung bei verschiedenen Malignitäten, zur Therapie der Malaria sowie von weiteren Krankheiten, bei denen eine Blockierung des Integrins VLA-4 und/oder eine Beeinflussung der Leukozytenaktivität zur Vorbeugung, Linderung oder Heilung angebracht erscheint.

Die Dosis bei der Anwendung der Verbindungen der Formel I kann innerhalb weiter Grenzen variieren und ist wie üblich in jedem einzelnen Fall den individuellen Gegebenheiten anzupassen. Sie hängt beispielsweise von der eingesetzten Verbindung oder von der Art und Schwere der zu behandelnden Krankheit ab oder davon, ob ein akuter oder chronischer Krankheitszustand behandelt wird oder ob Prophylaxe betrieben wird. Im allgemeinen ist bei der oralen Verabreichung eine Tagesdosis von etwa 0,01 bis 100 mg/kg, vorzugsweise 0,1 bis 10 mg/kg, insbesondere 0,3 bis 2 mg/kg (jeweils pro kg Körpergewicht) bei einem ca. 75 kg schweren Erwachsenen zur Erzielung wirksamer Ergebnisse angemessen. Bei intravenöser Applikation beträgt die Tagesdosis im allgemeinen etwa 0,01 bis 50 mg/kg, vorzugsweise 0,01 bis 10 mg/kg Körpergewicht. Die Tagesdosis kann, insbesondere bei der Applikation größerer Mengen, in mehrere, zum Beispiel 2, 3 oder 4, Teilverabreichungen aufgeteilt werden. Gegebenenfalls kann es, je nach individuellem Verhalten, erforderlich werden, von der angegebenen Tagesdosis nach oben oder nach unten abzuweichen.

Gegenstand der vorliegenden Erfindung sind auch die Verbindungen der Formel I zur Hemmung der Adhäsion und/oder Migration von Leukozyten oder zur Hemmung des VLA-4-Rezeptors und die Verwendung der Verbindungen der Formeln I zur Herstellung von Arzneimitteln dafür, also von Arzneimitteln zur Therapie oder Prophylaxe von Krankheiten, bei denen die Leukozytenadhäsion und/oder Leukozytenmigration ein unerwünschtes Ausmaß aufweist, oder von Krankheiten, bei denen VLA-4-abhängige Adhäsionsvorgänge eine Rolle spielen, sowie die Verwendung der Verbindungen der Formel und/oder ihrer physiologisch verträglichen Salze bei der Therapie und Prophylaxe derartiger Krankheiten.

Die Verbindungen der Formel I und ihre Salze können weiterhin für diagnostische Zwecke, zum Beispiel in in-vitro-Diagnosen, und als Hilfsmittel in biochemischen Untersuchungen eingesetzt werden, bei denen eine VLA-4-Blockierung oder eine Beeinflussung von Zell-Zell- oder Zell-Matrix-Interaktionen angestrebt wird. Weiterhin können sie als Zwischenprodukte für die Herstellung anderer Verbindungen dienen, insbesondere anderer Arzneimittelwirkstoffe, die aus den Verbindungen der Formeln I beispielsweise durch Abwandlung oder Einführung von Resten oder funktionellen Gruppen erhältlich sind.

### Beispiele

Die Verbindungen wurden über Massenspektren (MS) und/oder NMR-Spektren identifiziert. Verbindungen, die durch Chromatographie unter Verwendung eines Laufmittels gereinigt wurden, das beispielsweise Essigsäure oder Trifluoressigsäure enthielt, und anschließend gefriergetrocknet wurden, enthielten zum Teil je nach Durchführung der Gefriertrocknung noch die aus dem Laufmittel stammende Säure, sind also teilweise oder vollständig in Form eines Salzes der verwendeten Säure, zum Beispiel in Form des Essigsäuresalzes oder Tritluoressigsäuresalzes, angefallen.

Es bedeuten:
- DMF: N,N-Dimethylformamid
- THF: Tetrahydrofuran
- DCC: N,N'-Dicyclohexylcarbodiimid
- HOBt: 1-Hydroxybenzotriazol
- HOOBt: 3-Hydroxy-4-oxo-3,4-dihydro-1,2,3-benzotriazin

### Beispiel 1

### (S)-3-(((R,S)-4-(4-(Amino-imino-methyl)-phenyl)-3-benzyl-4-methyl-2,5-dioxoimidazolidin-1-yl)-acetylamino)-2-(1-adamantylmethyloxycarbonylamino)-propionsäure

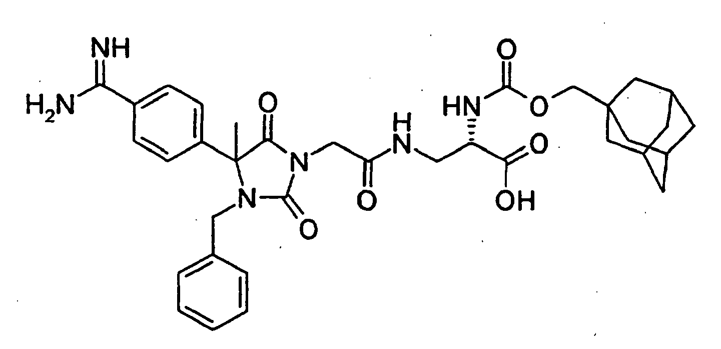

### 1a) (R,S)-4-(4-Cyanphenyl)-4-methyl-2,5-dioxoimidazolidin

20 g (138 mmol) p-Acetylbenzonitril, 115,6 g Ammoniumcarbonat (1,21 mol) und 11,6 g Kaliumcyanid (178 mmol) wurden in 600 ml einer Mischung aus 50 % Ethanol und 50 % Wasser gelöst. Das Gemisch wurde 5 Stunden bei 55 °C gerührt und über Nacht bei Raumtemperatur stehen gelassen. Die Lösung wurde mit 6 N Salzsäure auf einen pH-Wert von 6,3 eingestellt und anschließend zwei Stunden bei Raumtemperatur gerührt. Der Niederschlag wurde abgesaugt, mit Wasser gewaschen und über Phosphorpentoxid im Hochvakuum getrocknet. Ausbeute: 22,33 g (75 %).

### 1b) ((R,S)-4-(4-Cyanphenyl)-4-methyl-2,5-dioxoimidazolidin-1-yl)-essigsäure-methylester

1,068 g Natrium (46,47 mmol) wurden unter Stickstoff in 110 ml absolutem Methanol gelöst. Die klare Lösung wurde mit 10 g (R,S)-4-(4-Cyanphenyl)-4-methyl-2,5-dioxoimidazolin (46,47 mmol) versetzt und das Gemisch wurde 2 h unter Rückfluß gekocht. Man gab 7,75 g (46,68 mmol) Kaliumiodid zu und tropfte innerhalb einer Stunde eine Lösung von 4,53 ml Chloressigsäuremethylester (51,3 mmol) in 5 ml Methanol zu. Es wurde 6 Stunden zum Sieden erhitzt, über Nacht bei Raumtemperatur stehen gelassen und eingeengt. Der ölige Rückstand wurde über Kieselgel mit Methylenchlorid/Essigester (9:1) chromatographiert. Ausbeute: 8,81 g (66 %).

### 1c) ((R,S)-4-(4-Cyanphenyl)-3-benzyl-4-methyl-2,5-dioxoimidazolidin-1-yl)-essigsäure-methylester

Man gab zu einer Lösung von 4,5 g (15,7 mmol) von ((R,S)-4-(4-Cyanphenyl)-4-methyl-2,5-dioxoimidazolidin-1-yl)-essigsäure-methylester in 25 ml absolutem DMF unter Argon bei 0 °C 754 mg (17,27 mmol) Natriumhydrid zu, ließ 15 min bei Raumtemperatur rühren, gab 2,05 ml (17,27 mmol) Benzylbromid zu und ließ 4 h bei Raumtemperatur rühren. Das Lösungsmittel wurde im Vakuum entfernt und der Rückstand mit Heptan/Essigester (7:3) über Kieselgel chromatographiert. Nach Einengen der Produktfraktionen erhielt man 9,81 g (76 %) der Titelverbindung.

### 1d) ((R,S)-4-(4-Amino-imino-methyl)-phenyl)-3-benzyl-4-methyl-2,5-dioxoimidazolidin-1-yl)-essigsäure-methylester-Hydrochlorid

Eine Suspension von 4,42 g (11,7 mmol) an ((R,S)-4-(4-Cyanphenyl)-3-benzyl-4-methyl-2,5-dioxoimidazolidin-1-yl)-essigsäure-methylester in 80 ml absolutem Ethanol wurde auf 0 °C abgekühlt. Trockenes Chlorwasserstoff-Gas wurde in die Suspension eingeleitet, wobei die Temperatur stets unter 10 °C gehalten wurde, bis im IR-Spektrum die Nitrilbande nicht mehr vorlag. Die ethanolische Lösung wurde auf die Hälfte eingeengt und mit 1 l Diethylether versetzt. Die Suspension wurde im Vakuum eingeengt und der Rückstand am Hochvakuum getrocknet. Das so erhaltene Zwischenprodukt wurde in 60 ml absolutem Isopropanol gelöst und bei 50 °C mit 13,7 ml einer 1,9 N Lösung von Ammoniak in Isopropanol versetzt. Nach 5 h Rühren bei 50 °C wurde das Reaktionsgemisch abgekühlt und in 1 I Diethylether gegossen. Der Niederschlag wurde abgesaugt, das Filtrat eingeengt, beide Rückstände wurden vereinigt und durch Chromatographie über Kieselgel mit Dichlormethan/Methanol/Essigsäure/Wasser (9:1:0,1:0,1) gereinigt. Man erhielt 2,94 g (64 %) der Titelverbindung.

### 1e) ((R,S)-4-(4-Amino-imino-methyl)-phenyl)-3-benzyl-4-methyl-2,5-dioxoimidazolidin-1-yl)-essigsäure-Hydrochlorid

2,82 g (7,2 mmol) an ((R,S)-4-(4-Amino-imino-methyl)-phenyl)-3-benzyl-4-methyl-2,5-dioxoimidazolidin-1-yl)-essigsäure-methylester-Hydrochlorid wurden in 60 ml konz. Salzsäure 5 h unter Rückfluß erhitzt. Die Lösung wurde im Vakuum eingeengt, der Rückstand mit Wasser verdünnt und gefriergetrocknet. Man erhielt 1,885 g (63 %) der Titelverbindung.

### 1f) (S)-3-Amino-2-benzyloxycarbonylaminopropionsäure-tert-butylester

10 g (42 mmol) (S)-3-Amino-2-benzyloxycarbonylamino-propionsäure wurden in einem Gemisch aus 100 ml Dioxan, 100 ml Isobutylen und 8 ml konz, H₂SO₄ 3 Tage bei 20 atm N₂-Druck im Autoklaven geschüttelt. Überschüssiges Isobutylen wurde abgeblasen und zur verbleibenden Lösung wurden 150 ml Diethylether und 150 ml gesättigte NaHCO₃-Lösung gegeben. Die Phasen wurden getrennt und die wäßrige Phase wurde 2 x mit je 100 ml Diethylether extrahiert. Die vereinigten organischen Phasen wurden 2 x mit je 100 ml Wasser gewaschen und über Na₂SO₄ getrocknet. Nach Entfemen des Lösungsmittels im Vakuum erhielt man 9,58 g (78 %) der Titelverbindung als blaßgelbes Öl.

### 1 g) (S)-2-Benzyloxycarbonylamino-3-tert-butoxycarbonylamino-propionsäure-tert-butylester

Man gab zu einer Lösung von 10 g (34 mmol) (S)-3-Amino-2-benzyloxycarbonylaminopropionsäure-tert-butylester in 600 ml THF/Wasser (2:1) bei 0 °C 8,9 g (40,8 mmol) Di-tert-butyl-dicarbonat und anschließend portionsweise 1 N NaOH, so daß der pH der Lösung zwischen 9 und 10 lag (Verbrauch an 1 N NaOH: 32 ml). Nach 3 h Rühren bei Raumtemperatur gab man 1 I Wasser zu und extrahierte 3 mal mit Diethylether. Nach Trocknen über Natriumsulfat, Filtration und Entfernen des Lösungsmittels im Vakuum wurde der Rückstand mit Dichlormethan/Methanol (20:1) über Kieselgel chromatographiert. Man erhielt 13,19 g (98 %) der Titelverbindung.

### 1h)(S)-2-Amino-3-tert-butoxycarbonylamino-propionsäure-tert-butylester-Hydrochlorid

13,1 g (S)-2-Benzyloxycarbonylamino-3-tert-butoxycarbonylamino-propionsäure-tert-butylester wurden in Methanol/HCl über 10 % Pd/C hydriert. Nach 1,5 h wurde filtriert und das Filtrat im Vakuum eingeengt. Man erhielt 9,77 g (99 %) der Titelverbindung als farblosen Feststoff.

### 1i) (S)-2-(1-Adamantylmethyloxycarbonylamino)-3-tert-butoxycarbonylamino-propionsäure-tert-butylester

Eine Lösung von 10,9 g (65,4 mmol) 1-(Hydroxymethyl)-adamantan und 10,6 g (65,4 mmol) Carbonyldiimidazol in 60 ml THF wurde 1,5 h bei 50°C gerührt. Man gab 9,7 g (32,7 mmol) (S)-2-Amino-3-tert-butoxycarbonylamino-propionsäure-tert-butylester-Hydrochlorid in 25 ml THF und 5,6 ml (32,7 mmol) Diisopropyl-ethylamin zu, rührte 4 h bei 60°C und ließ über Nacht bei Raumtemperatur stehen. Das Lösungsmittel wurde im Vakuum entfernt und der Rückstand mit Heptan/Essigester (7:3) über Kieselgel chromatographiert. Man erhielt 8,7 g (59 %) der Titelverbindung als farbloses Öl.

### 1j) (S)-2-(1-Adamantylmethyloxycarbonylamino)-3-amino-propionsäure-tert-butylester

Eine Lösung von 8,7 g (19,22 mmol) (S)-2-(1-Adamantylmethyloxycarbonylamino)-3-tert-butoxycarbonylamino-propionsäure-tert-butylester in 180 ml Trifluoressigsäure/Dichlormethan (1:1) wurde nach 1 min in 1,5 I eiskalte NaHCO₃-Lösung gegeben, das Gemisch dreimal mit Dichlormethan extrahiert und die vereinigten Dichlormethan-Phasen anschließend über Natriumsulfat getrocknet.

Nach Filtration und Entfemen des Lösungsmittels im Vakuum erhielt man 6,35 g (94 %) der Titelverbindung als farblosen Feststoff.

### 1k) (S)-3-(((R,S)-4-(4-(Amino-imino-methyl)-phenyl)-3-benzyl-4-methyl-2,5-dioxoimidazolidin-1-yl)-acetylamino)-2-(1-adamantylmethyloxycarbonylamino)-propionsäure

417 mg (1 mmol) ((R,S)-4-(4-(Amino-imino-methyl)phenyl)-3-benzyl-4-methyl-2,5-dioxoimidazolidin-1-yl)-essigsäure-Hydrochlorid und 163 mg (1 mmol) HOOBt wurden in 5 ml DMF suspendiert und bei 0 °C mit 220 mg (1,1 mmol) DCC versetzt. Man rührte 1 h bei 0 °C und 1 h bei Raumtemperatur und gab anschließend 353 mg (1 mmol) (S)-2-(1-Adamantylmethyloxycarbonylamino)-3-amino-propionsäure-tert-butylester und 11,7 µl (0,9 mmol) N-Ethyl-morpholin zu, rührte 2 h bei Raumtemperatur und ließ über Nacht bei Raumtemperatur stehen. Nach Filtration wurde das Filtrat im Vakuum eingeengt und der Rückstand mit Dichlormethan/Methanol/Eisessig/Wasser (9:1:0,1:0,1) über Kieselgel chromatographiert. Nach Einengen der Produktfraktionen wurde der Rückstand in 4 ml 90 %iger Trifluoressigsäure gelöst und 1 h bei Raumtemperatur gerührt. Die Trifluoressigsäure wurde im Vakuum entfernt, der Rückstand zwischen Diethylether und Wasser verteilt, die Wasserphase eingeengt und der Rückstand durch Chromatographie über Kieselgel mit Dichlormethan/Methanol/EisessiglWasser (9:1:0,1:0,1) und anschließende präparative HPLC über RP-18 gereinigt. Nach Einengen der Produktfraktionen und Gefriertrocknen erhielt man 26,3 mg (4 %) der Titelverbindung.
FAB-MS: 659,4 (M+H)⁺

### Beispiel 2

### (S)-3-((S)-4-(4-(Amino-hydroximino-methyl)-phenyl)-3-(2-naphthyl-methyl)-4-methyl-2,5-dioxoimidazolidin-1-yl)-acetylamino)-2-(1-adamantylmethyloxycarbonylamino)-propionsäure

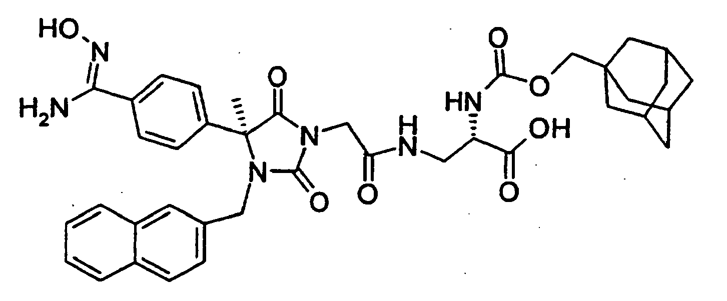

### 2a) ((S)-4-(4-Cyanphenyl)-3-(2-naphthyl-methyl)-4-methyl-2,5-dioxoimidazolidin-1-yl)-essigsäure-(2-naphthyl-methyl)-ester

Man gab zu einer Lösung von 13,66 g (50 mmol) ((S)-4-(4-Cyanphenyl)-4-methyl-2,5-dioxoimidazolidin-1-yl)-essigsäure in 100 ml absolutem DMF unter Eiskühlung 5,28 g (110 mmol) Natriumhydrid. Nach 1 h Rühren bei Raumtemperatur wurden innerhalb von 1 h 24,3 g (110 mmol) 2-Brommethyl-naphthalin zugegeben. Das Reaktionsgemisch wurde 20 h bei Raumtemperatur gerührt und dann in ein Gemisch von Essigester/Wasser gegossen. Nach Phasentrennung wurde die Wasserphase mit Essigester extrahiert. Die vereinigten organischen Phasen wurden zweimal mit Wasser gewaschen und über Magnesiumsulfat getrocknet. Nach Filtration und Entfemen des Lösungsmittels im Vakuum wurde der Rückstand mit Heptan/Essigester (2:1) über Kieselgel chromatographiert. Man erhielt 8,51 g (56 %) der Titelverbindung.

### 2b) ((S)-4-(4-(Amino-hydroximino-methyl)-phenyl)-3-(2-naphthyl-methyl)-4-methyl-2,5-dioxoimidazolidin-1-yl)-essigsäure-(2-naphthyl-methyl)-ester

Man gab zu einer Lösung von 6,64 g (12 mmol) an ((S)-4-(4-Cyanphenyl)-3-(2-naphthyl-methyl)-4-methyl-2,5-dioxoimidazolidin-1-yl)-essigsäure-(2-naphthylmethyl)-ester in 120 ml absolutem Ethanol 1,67 g (24 mmol) Hydroxylammoniumchlorid und 5,04 ml (36 mmol) Triethylamin und erhitzte das Gemisch 2,5 h unter Rückfluß. Das Lösungsmittel wurde im Vakuum entfernt und der Rückstand zwischen Essigester und Wasser verteilt. Die Phasen wurden getrennt und die Wasserphase mit Essigester extrahiert. Die vereinigten organischen Phasen wurden mit Wasser gewaschen und über Magnesiumsulfat getrocknet. Nach Filtration wurde das Lösungsmittel im Vakuum entfernt und man erhielt 6,08 g (86 %) an der Titelverbindung.

### 2c) ((S)-4-(4-(Amino-hydroximino-methyl)-phenyl)-3-(2-naphthyl-methyl)-4-methyl-2,5-dioxoimidazolidin-1-yl)-essigsäure

2 g (3,4 mmol) ((S)-4-(4-(Amino-hydroximino-methyl)-phenyl)-3-(2-naphthyl-methyl)-4-methyl-2,5-dioxoimidazolidin-1-yl)-essigsäure-(2-naphthyl-methyl)-ester in 200 ml absolutem Methanol wurden 4 h über Palladiumhydroxid/Bariumsulfat hydriert. Der Katalysator wurde abfiltriert, das Filtrat im Vakuum eingeengt und der Rückstand mit Essigester verrührt. Das Produkt wurde abgesaugt und im Hochvakuum getrocknet. Man erhielt 0,56 g (37 %) der Titelverbindung.

### 2d) (S)-3-((S)-4-(4-(Amino-hydroximino-methyl)-phenyl)-3-(2-naphthyl-methyl)-4-methyl-2,5-dioxoimidazolidin-1-yl)-acetylamino)-2-(1-adamantylmethyloxycarbonylamino)-propionsäure-tert-butylester

Zu einer Lösung von 223 mg (0,5 mmol) ((S)-4-(4-(Amino-hydroximino-methyl)-phenyl)-3-(2-naphthyl-methyl)-4-methyl-2,5-dioxoimidazolidin-1-yl)-essigsäure und 176 mg (0,5 mmol) (S)-2-(1-Adamantylmethyloxycarbonylamino)-3-amino-propionsäure-tert-butylester (siehe Beispiel 1) in 10 ml absolutem DMF wurden 164 mg (0,5 mmol) TOTU (O-(Cyan(ethoxycarbonyl)methylenamino)-1,1,3,3-tetramethyluronium-tetrafluoroborat) und 165 mg (1,26 mmol) Diisopropyl-ethylamin gegeben. Nach 2 h Rühren bei Raumtemperatur und Stehen über Nacht wurde das Reaktionsgemisch im Vakuum eingeengt und der Rückstand zwischen Essigester und Wasser verteilt. Die Phasen wurden getrennt, die Wasserphase mit Essigester extrahiert und die vereinigten organischen Phasen wurden mit gesättigter NaHCO₃-Lösung und Wasser gewaschen. Nach Trocknen über Natriumsulfat, Filtration und Einengen des Filtrats im Vakuum wurde der Rückstand in Essigester aufgenommen und die Lösung nacheinander mit KHSO₄/K₂SO₄-Lösung, gesättigter NaHCO₃-Lösung und Wassser gewaschen und über Magnesiumsulfat getrocknet. Man erhielt 240 mg (62 %) der Titelverbindung.

### 2e) (S)-3-((S)-4-(4-(Amino-hydroximino-methyl)-phenyl)-3-(2-naphthyl-methyl)-4-methyl-2,5-dioxoimidazolidin-1-yl)-acetylamino)-2-(1-adamantylmethyloxycarbonylamino)-propionsäure

220 mg (0,28 mmol) (S)-3-((S)-4-(4-(Amino-hydroximino-methyl)-phenyl)-3-(2-naphthyl-methyl)-4-methyl-2,5-dioxoimidazolidin-1-yl)-acetylamino)-2-(1-adamantylmethyloxycarbonylamino)-propionsäure-tert-butylester wurden in 20 ml 90 %iger Trifluoressigsäure gelöst. Nach 1 h bei Raumtemperatur wurde die Trifluoressigsäure im Vakuum entfernt und der Rückstand mit Diethylether verrührt. Das Produkt wurde abgesaugt, mit Diethylether gewaschen und im Hochvakuum getrocknet. Man erhielt 110 mg (54 %) der Titelverbindung (als Trifluoressigsäuresalz).
ES(+)-MS: 725,4 (M+H)⁺

### Beispiel 3 (Zwischenprodukt)

### (S)-3-(((R,S)-4-(4-(Amino-iminomethyl)-phenyl)-3-benzyl-4-methyl-2,5-dioxoimidazolidin-1-yl)-acetylamino)-2-amino-propionsäure-Dihydrochlorid

Eine Lösung von 4,4 g (6,7 mmol) (S)-3-(((R,S)-4-(4-(Amino-imino-methyl)-phenyl)-3-benzyl-4-methyl-2,5-dioxoimidazolidin-1-yl)-acetylamino)-2-benzyloxycarbonylamino-propionsäure (hergestellt durch Kupplung von ((R,S)-4-(4-Amino-imino-methyl)-phenyl)-3-benzyl-4-methyl-2,5-dioxoimidazolidin-1-yl)-essigsäure-Hydrochlorid und (S)-3-Amino-2-benzyloxycarbonylamino-propionsäure-tert-butylester analog Beispiel 1 und anschließende Spaltung des tert-Butylesters analog Beispiel 1) in 100 ml Methanol wurde über Palladiumhydroxid/Bariumsulfat 1 h bei Raumtemperatur hydriert. Der Katalysator wurde abfiltriert, das Lösungsmittel im Vakuum entfernt und der Rückstand in 40 ml 6 N Salzsäure 30 min bei 40 °C gerührt. Die Lösung wurde im Vakuum eingeengt, mit Wasser verdünnt und gefriergetrocknet. Man erhielt 2,39 g (77 %) der Titelverbindung.

### Beispiel 5

### ((R,S)-4-(4-(Amino-imino-methyl)-phenyl)-3-(2-naphthyl-methyl)-4-methyl-2,5-dioxoimidazolidin-1-yl)-acetyl-L-(N-methyl-aspartyl)-L-valin-morpholid

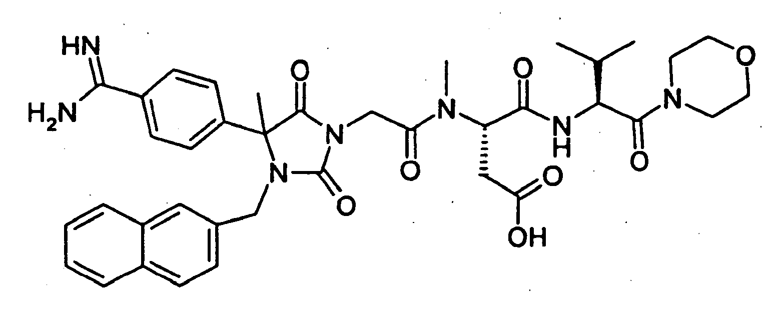

### 5a) L-Valin-morpholid

Man gab zu einer Lösung von 3,01 g (12 mmol) N-Benzyloxycarbonyl-L-valin und 1,04 g (12 mmol) Morpholin in 30 ml absolutem DMF 3,93 g (12 mmol) TOTU (siehe Beispiel 2) und 2,04 ml Diisopropyl-ethylamin. Nach 2 h Rühren bei Raumtemperatur wurde das Lösungsmittel im Vakuum entfernt, der Rückstand in Essigester aufgenommen und die Lösung nacheinander 3 x mit einer wäßrigen KHSO₄/K₂SO₄-Lösung, 3 x mit einer gesättigten NaHCO₃-Lösung und 3 x mit Wasser gewaschen. Nach Trocknen über Natriumsulfat, Filtration und Entfernen des Lösungsmittels im Vakuum erhielt man 3,88 g N-Benzyloxycarbonyl-L-valin-morpholid als Rohprodukt, das zur Abspaltung der Benzyloxycarbonylgruppe in Methanol 3 h über 10 % Pd/C hydriert wurde. Man erhielt 2,11 g (95 %) der Titelverbindung.

### 5b) L-(N-Methyl-aspartyl(OtBu))-L-valin-morpholid

Die Verbindung wurde durch Kupplung von L-Z-N(CH₃)-Asp(OtBu)-OH mit L-Valin-morpholid und anschließende hydrogenolytische Spaltung der Z-Gruppe (Benzyloxycarbonyl-Gruppe) wie unter a) beschrieben hergestellt. Aus 1,39 g (7,5 mmol) L-Valin-morpholid wurden so 2,4 g (86 %) der Titelverbindung erhalten.

### 5c) ((R,S)-4-(4-(Amino-imino-methyl)-phenyl)-3-(2-naphthyl-methyl)-4-methyl-2,5-dioxoimidazolidin-1-yl)-acetyl-L-(N-methyl-aspartyl)-L-valin-morpholid

Die Verbindung wurde durch Kupplung von ((R,S)-4-(4-(Amino-imino-methyl)-phenyl)-3-(2-naphthyl-methyl)-4-methyl-2,5-dioxoimidazolidin-1-yl)-essigsäure-Hydrochlorid (hergestellt analog Beispiel 1 unter Verwendung von 2-Brommethyl-naphthalin an Stelle von Benzylbromid) und L-(N-Methyl-aspartyl(OtBu))-L-valin-morpholid und nachfolgende Spaltung des tert-Butylesters mit 90%iger Trifluoressigsäure analog Beispiel 2 erhalten.
ES(+)-MS: 728,4 (M+H)⁺

Analog zur Verbindung des Beispiels 1 können weitere Carbamate erhalten werden, beispielsweise die (S)-3-(((S)-4-(4-(Amino-imino-ethyl)-phenyl)-3-benzyl-4-methyl-2,5-dioxoimidazolidin-1-yl)-((R,S)-2-(2-methylpropyl)-acetylamino)-2-(cyclohexylmethyloxycarbonylamino)-propionsäure der Formel, 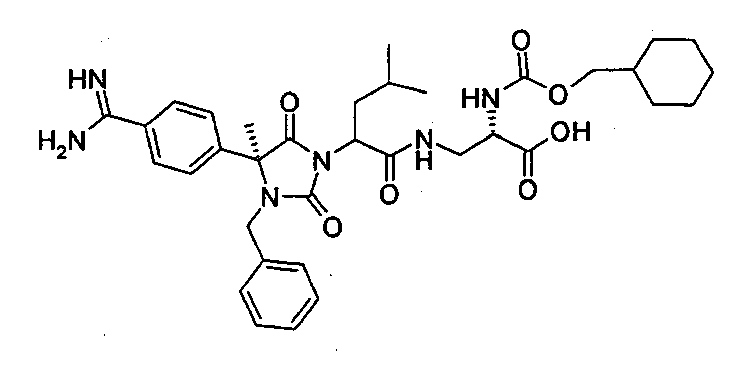 wobei an Stelle des im Beispiel 1 im Schritt b) eingesetzten Chloressigsäureesters beispielsweise ein 2-Brom-4-methylpentansäureester eingesetzt werden kann.

### Untersuchung der biologischen Aktivität

Als Testmethode für die Wirksamkeit der Verbindungen der Formel I auf die Interaktion zwischen VCAM-1 und VLA-4 wird ein Assay verwendet, der für diese Interaktion spezifisch ist. Die zellulären Bindungspartner, das heißt die VLA-4-Integrine, werden in ihrer natürlichen Form als Oberflächenmoleküle auf humanen U937-Zellen (ATCC CRL 1593), die zur Gruppe der Leukozyten gehören, angeboten. Als spezifische Bindungspartner werden gentechnisch hergestellte rekombinante lösliche Fusionsproteine, bestehend aus der extrazytoplasmatischen Domäne von humanen VCAM-1 und der konstanten Region eines humanen Immunglobulins der Subklasse IgG1, verwendet.

### Testmethode

### Assay zur Messung der Adhäsion von U937-Zellen (ATCC CRL 1593) an hVCAM-1 (1-3)-IgG

### 1. Herstellung von humanem VCAM-1(1-3)-IgG und humanem CD4-IgG

Eingesetzt wurde ein genetisches Konstrukt zur Expression der extrazellulären Domäne des humanen VCAM-1, verbunden mit der genetischen Sequenz der schweren Kette des humanen Immunglobulins IgG1 (Hinge, CH2 und CH3 Regionen), von Dr. Brian Seed, Massachusetts General Hospital, Boston, USA (vgl. Damle und Aruffo, Proc. Natl. Acad. Sci. USA 1991, 88, 6403-6407). Das lösliche Fusionsprotein hVCAM-1(1-3)-IgG enthielt die drei aminoterminalen extrazellulären Immunglobulin-ähnlichen Domänen des humanen VCAM-1 (Damle und Aruffo, Proc. Natl. Acad. Sci. USA 1991, 88, 6403). CD4-IgG (Zettlmeissl et al., DNA and Cell Biology 1990, 9, 347) diente als Fusionsprotein für negative Kontrollen. Die rekombinanten Proteine wurden als lösliche Proteine nach DEAE/Dextranvermittelter DNA-Transfektion in COS-Zellen (ATCC CRL1651) gemäß Standardprozeduren exprimiert (Ausubel et al., Current Protocols in Molecular Biology, John Wiley & Sons, Inc., 1994).

### 2. Assay zur Messung der Adhäsion von U937-Zellen an hVCAM-1 (1-3)-IgG

2.1 96 well-Mikrotitertestplatten (Nunc Maxisorb) wurden mit 100 µl/well einer Ziege-anti-human-IgG-Antikörperlösung (10 µg/ml in 50 mM Tris, pH 9,5) 1 Stunde bei Raumtemperatur inkubiert. Nach Entfemen der Antikörperlösung wurde einmal mit PBS gewaschen.
2.2 150 µ/well eines Blockierungspuffers (1% BSA in PBS) wurde 0,5 Stunden bei Raumtemperatur auf den Platten inkubiert. Nach Entfemen des Blockierungspuffers wurde einmal mit PBS gewaschen.
2.3 100 µl pro well eines Zellkulturüberstandes von transfektierten COS-Zellen wurde für 1,5 Stunden bei Raumtemperatur auf den Platten inkubiert. Die COS-Zellen waren mit einem Plasmid transfiziert, welches für die drei N-terminalen Immunglobulin-ähnlichen Domänen des VCAM-1, gekoppelt an den Fc-Teil von humanem IgG₁ (hVCAM-1(1-3)-IgG), codiert. Der Gehalt an hVCAM-1(1-3)-IgG betrug ca. 0,5 - 1 µg/ml. Nach Entfernen des Kulturüberstandes wurde einmal mit PBS gewaschen.
2.4 Die Platten wurden mit 100 µl/well Fc-Rezeptor-Blockpuffer (1 mg/ml γ-Globulin, 100 mM NaCl, 100 µM MgCl₂, 100 µM MnCl₂, 100 µM CaCl₂, 1 mg/ml BSA in 50 mM HEPES, pH 7,5) für 20 Minuten bei Raumtemperatur inkubiert. Nach Entfernen des Fc-Rezeptor-Blockpuffers wurde einmal mit PBS gewaschen.
2.5 20 µl Bindungspuffer (100 mM NaCl, 100 µM MgCl₂, 100 µM MnCl₂, 100 µM CaCl₂, 1 mg/ml BSA in 50 mM HEPES, pH 7,5) wurden vorgelegt, die zu testenden Substanzen in 10 µl Bindungspuffer zugegeben und für 20 Minuten inkubiert. Als Kontrollen dienten Antikörper gegen VCAM-1 (BBT, Nr. BBA6) und gegen VLA-4 (Immunotech, Nr. 0764).
2.6 U937-Zellen wurden 20 Minuten in Fc-Rezeptor-Blockpuffer inkubiert und anschließend in einer Konzentration von 1 x 10⁶/ml und in einer Menge von 100 µl pro well zupipettiert (Endvolumen 125µl/well).
2.7 Die Platten wurden in einem 45°-Winkel in Stoppuffer (100 mM NaCl, 100 µM MgCl₂, 100 µM MnCl₂, 100 µM CaCl₂ in 25 mM Tris, pH 7,5) langsam eingetaucht und aufgeschlagen. Der Vorgang wurde wiederholt.
2.8 Anschließend wurden 50 µl/well einer Färbelösung (16,7 µg/ml Hoechst Farbstoff 33258, 4 % Formaldehyd, 0,5 % Triton-X-100 in PBS) 15 Minuten auf den Platten inkubiert.
2.9 Die Platten wurden ausgeschlagen, in einem 45°-Winkel in Stoppuffer (100 mM NaCl, 100 µM MgCl₂, 100 µM MnCl₂, 100 µM CaCl₂ in 25 mM Tris, pH 7,5) langsam eingetaucht. Der Vorgang wurde wiederholt. Anschließend wurde mit der Flüssigkeit in einem Cytofluorimeter (Millipore) gemessen (Sensitivität: 5, Filter: Anregungswellenlänge: 360 nm, Emissionswellenlänge: 460 nm).

Die Intensität des von den angefärbten U937-Zellen emittierten Lichts ist ein Maß für die Zahl der an der Platte verbliebenen, an das hVCAM-1(1-3)-IgG adhärierten U937-Zellen und somit ein Maß für die Fähigkeit der zugesetzten Testsubstanz, diese Adhäsion zu hemmen. Aus der Hemmung der Adhäsion bei verschiedenen Konzentrationen der Testsubstanz wurde die Konzentration IC₅₀ berechnet, die zu einer Hemmung der Adhäsion um 50% führt.

Es wurden die folgenden Testergebnisse erhalten:

| Beispiel | U937NCAM-1 Zelladhäsionstest |
|---|---|
| | IC₅₀ (µM) |
| 1 | 1,25 |
| 5 | 0,73 |

## Patentansprüche

1. Verbindung der Formel I, 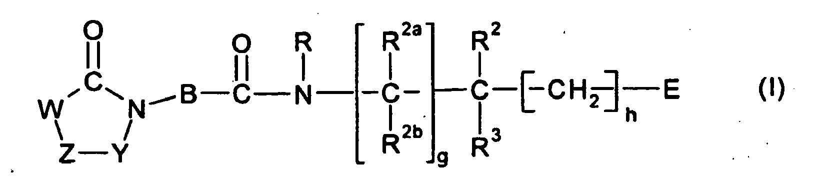 in der
W für R¹-A-C(R¹³) und darin A für den zweiwertigen Phenylenrest steht;
Y für eine Carbonylgruppe steht;
Z für N(R⁰) steht;
B für einen zweiwertigen Methylenrest steht, der unsubstituiert ist oder durch einen (C₁-C₆)-Alkylrest substituiert ist;
E für R¹⁰CO steht;
R und R^{b} unabhängig voneinander für Wasserstoff oder (C₁-C₄)-Alkyl stehen;
R⁰ für im Arylrest gegebenenfalls substituiertes (C₆-C₁₂)-Aryl-(C₁-C₂)-alkyl steht;
R¹ für H₂N-C(=NH), H₂N-C(=N-OH), CH₃O-CO-NH-C(=NH), H₂N-C(=NH)-NH oder
H₂N-CH₂ steht;
R², R^{2a} und R^{2b} für Wasserstoff stehen;
R³ für R¹¹NH oder CO-R⁵-Het steht;
R⁵ für den zweiwertigen Rest einer natürlichen oder unnatürlichen α-Aminosäure mit einer lipophilen Seitenkette steht, wobei freie funktionelle Gruppen durch in der Peptidchemie übliche Schutzgruppen geschützt sein können oder als Ester oder Amide vorliegen können, und wobei das Stickstoffatom der N-terminalen Aminogruppe einen Rest R^{b} trägt;
R¹⁰ für Hydroxy, (C₁-C₄)-Alkoxy, Phenoxy, Benzyloxy oder (C₁-C₄)-Alkylcarbonyloxy-(C₁-C₄)-alkoxy steht;
R¹¹ für R¹⁵O-CO oder R¹⁵S(O)₂ steht;
R¹³ für (C₁-C₆)-Alkyl steht;
R¹⁵ für R¹⁶-(C₁-C₃)-alkyl oder für R¹⁶ steht;
R¹⁶ für den Rest eines 5-gliedrigen bis 6-gliedrigen monocyclischen Ringes oder für den Rest eines 7-gliedrigen bis 12-gliedrigen bicyclischen oder tricyclischeh Ringes steht, wobei diese Ringe gesättigt sind und auch durch einen oder zwei gleiche oder verschiedene (C₁-C₄)-Alkylreste substituiert sein können;
Het für den Rest eines über ein Ring-Stickstoffatom gebundenen 5-gliedrigen oder 6-gliedrigen monocyclischen Heterocyclus steht, der gesättigt, ist und der gegebenenfalls ein zusätzliches Ring-Heteratom aus der Reihe Sauerstoff und Schwefel enthalten kann und der durch einen oder zwei gleiche oder verschiedene (C₁-C₄)-Alkylreste substituiert sein kann;
g und h unabhängig voneinander für 0 oder 1 stehen;
in allen ihren stereoisomeren Formen und Mischungen davon in allen Verhältnissen, und ihre physiologisch verträglichen Salze.

2. Verbindung der Formel I gemäß Anspruch 1, in der
W für R¹-A-C(R¹³) und darin A für den zweiwertigen Phenylenrest steht;
Y für eine Carbonylgruppe steht;
Z für IV(R⁰) steht;
B für einen zweiwertigen Methylenrest steht, der unsubstituiert ist oder durch einen (C₁-C₆)-Alkylrest substituiert ist;
E für R¹⁰CO steht;
R und R^{b} unabhängig voneinander für Wasserstoff oder Methyl stehen;
R⁰ für im Arylrest gegebenenfalls substituiertes (C₆-C₁₂)-Aryl-(C₁-C₂)-alkyl steht;
R¹ für H₂N-C(=NH), H₂N-C(=NOH), CH₃O-CO-NH-C(=NH), H₂N-C(=NH)-NH oder H₂N-CH₂ steht;
R², R^{2a} und R^{2b} für Wasserstoff stehen;
R³ für R¹¹NH oder CO-R⁵-Het steht;
R⁵ für den zweiwertigen Rest einer natürlichen oder unnatürlichen α-Aminosäure mit einer lipophilen Seitenkette steht, wobei freie funktionelle Gruppen durch in der Peptidchemie übliche Schutzgruppen geschützt sein können oder als Ester oder Amide vorliegen können, und wobei das Stickstoffatom der N-terminalen Aminogruppe einen Rest R^{b} trägt;
R¹⁰ für Hydroxy, (C₁-C₄)-Alkoxy, Phenoxy, Benzyloxy oder (C₁-C₄)-Alkylcarbonyloxy-(C₁-C₄)-alkoxy steht;
R¹¹ für R¹⁵O-CO steht;
R¹³ für (C₁-C₆)-Alkyl steht;
R¹⁵ für R¹⁶ oder R¹⁶-CH₂ steht;
R¹⁶ für Cyclopentyl, Cyclohexyl, 1-Adamantyl, 2-Adamantyl oder Noradamantyl steht;
Het für den Rest eines über ein Ring-Stickstoffatom gebundenen 5-gliedrigen oder 6-gliedrigen monocyclischen Heterocyclus steht, der gesättigt ist und der gegebenenfalls ein Sauerstoffatom als zusätzliches Ring-Heteratom enthalten kann und der durch einen oder zwei gleiche oder verschiedene (C₁-C₄)-Alkylreste substituiert sein kann;
g und h unabhängig voneinander für 0 oder 1 stehen;
in allen ihren stereoisomeren Formen und Mischungen davon in allen Verhältnissen, und ihre physiologisch verträglichen Salze.

3. Verbindung der Formel I gemäß Anspruch 1 und/oder 2, in der R³ für R¹¹NH steht, g für 1 steht und h für 0 steht, in allen ihren stereoisomeren Formen und Mischungen davon in allen Verhältnissen, und ihre physiologisch verträglichen Salze.

4. Verbindung der Formel I gemäß einem oder mehreren der Ansprüche 1 bis 3, in der R¹⁶ für 1-Adamantyl oder 2-Adamantyl steht, in allen ihren stereoisomeren Formen und Mischungen davon in allen Verhältnissen, und ihre physiologisch verträglichen Salze.

5. Verbindung der Formel I gemäß Anspruch 1 und/oder 2, in der R³ für CO-R⁵-Het steht, g für 0 steht und h für 1 steht, in allen ihren stereoisomeren Formen und Mischungen davon in allen Verhältnissen, und ihre physiologisch verträglichen Salze.

6. Verbindung der Formel I gemäß einem oder mehreren der Ansprüche 1 bis 5, in der R¹³ für Methyl steht, in allen ihren stereoisomeren Formen und Mischungen davon in allen Verhältnissen, und ihre physiologisch verträglichen Salze.

7. Verfahren zur Herstellung einer Verbindung der Formel I gemäß einem oder mehreren der Ansprüche 1 bis 6, **dadurch gekennzeichnet, daß** man eine Fragmentkondensation einer Verbindung der Formel II 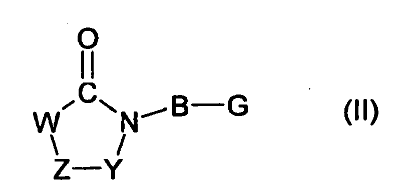 mit einer Verbindung der Formel III 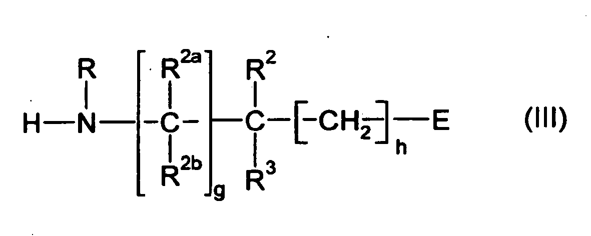 durchführt, wobei in den Formeln II und III die Gruppen W, Y, Z, B, E, R, R², R^{2a}, R^{2b} und R³ sowie g und h wie in den Ansprüchen 1 bis 6 definiert sind oder auch funktionelle Gruppen in geschützter Form oder in Form von Vorstufen enthalten sein können, und wobei G für Hydroxycarbonyl, (C₁-C₆)-Alkoxycarbonyl oder ein aktiviertes Carbonsäurederivat steht.

8. Verbindung der Formel I gemäß einem oder mehreren der Ansprüche 1 bis 6 und/oder ihre physiologisch verträglichen Salze zur Verwendung als Arzneimittel.

9. Pharmazeutisches Präparat, **dadurch gekennzeichnet, daß** es eine oder mehrere Verbindungen der Formel I gemäß einem oder mehreren der Ansprüche 1 bis 6 und/oder ihre physiologisch verträglichen Salze neben pharmazeutisch einwandfreien Trägerstoffen und/oder Zusatzstoffen enthält.

10. Verbindung der Formel I gemäß einem oder mehreren der Ansprüche 1 bis 6 und/oder ihre physiologisch verträglichen Salze zur Verwendung als Entzündungshemmstoffe.

11. Verbindung der Formel I gemäß einem oder mehreren der Ansprüche 1 bis 6 und/oder ihre physiologisch verträglichen Salze zur Verwendung in der Therapie oder Prophylaxe der rheumatoiden Arthritis, der inflammatorischen bowel disease, des systemischen Lupus erythematosus oder von inflammatorischen Erkrankungen des zentralen Nervensystems.

12. Verbindung der Formel I gemäß einem oder mehreren der Ansprüche 1 bis 6 und/oder ihre physiologisch verträglichen Salze zur Verwendung in der Therapie oder Prophylaxe von Asthma oder Allergien.

13. Verbindung der Formel I gemäß einem oder mehreren der Ansprüche 1 bis 6 und/oder ihre physiologisch verträglichen Salze zur Verwendung in der Therapie oder Prophylaxe von cardiovaskulären Erkrankungen, der Arteriosklerose, von Restenosen oder von Diabetes, zur Verhinderung der Schädigung von Organtransplantaten, zur Hemmung von Tumorwachstum oder Tumormetastasierung oder zur Therapie der Malaria.

14. Verbindung der Formel I gemäß einem oder mehreren der Ansprüche 1 bis 6 und/oder ihre physiologisch verträglichen Salze zur Verwendung als Hemmstoffe der Adhäsion und/oder der Migration von Leukozyten oder als Hemmstoffe des VLA-4-Rezeptors.

## Claims

1. A compound of the formula I 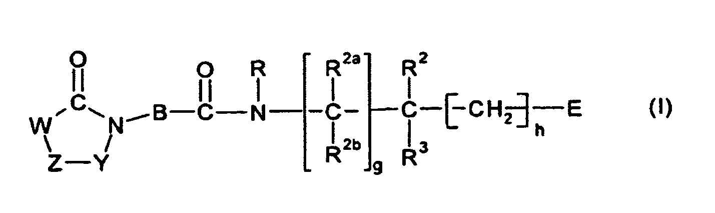 in which
W is R¹-A-C(R¹³) and therein A is the divalent phenylene radical;
Y is a carbonyl group;
Z is N(R⁰);
B is a divalent methylene radical which is unsubstituted or is substituted by a (C₁-C₆)-alkyl radical;
E is R¹⁰CO;
R and R^{b} are independently of one another hydrogen or (C₁-C₄)-alkyl;
R⁰ is (C₆-C₁₂)-aryl-(C₁-C₂)-alkyl which is optionally substituted in the aryl radical;
R¹ is H₂N-C(=NH), H₂N-C(=N-OH), CH₃O-CO-NH-C(=NH), H₂N-C(=NH)-NH or H₂N-CH₂;
R², R^{2a} and R^{2b} are hydrogen;
R³ is R¹¹NH or CO-R⁵-Het;
R⁵ is the divalent radical of a natural or unnatural α-amino acid having a lipophilic side chain, where free functional groups may be protected by protective groups customary in peptide chemistry or may be in the form of esters or amides, and where the nitrogen atom of the N-terminal amino group carries a radical R^{b};
R¹⁰ is hydroxy, (C₁-C₄)-alkoxy, phenoxy, benzyloxy or (C₁-C₄)-alkylcarbonyloxy-(C₁-C₄)-alkoxy;
R¹¹ is R¹⁵O-CO or R¹⁵S(O)₂;
R¹³ is (C₁-C₆)-alkyl;
R¹⁵ is R¹⁶-(C₁-C₃)-alkyl or is R¹⁶;
R¹⁶ is the radical of a 5-membered to 6-membered monocyclic ring or is the radical of a 7-membered to 12-membered bicyclic or tricyclic ring, where these rings are saturated and may also be substituted by one or two identical or different (C₁-C₄)-alkyl radicals;
Het is the radical of a 5-membered or 6-membered monocyclic heterocycle which is bonded via a ring nitrogen atom, which is saturated and which may optionally comprise an additional ring heteroatom from the series oxygen and sulfur, and which may be substituted by one or two identical or different (C₁-C₄)-alkyl radicals;
g and h are independently of one another 0 or 1;
in all its stereoisomeric forms and mixtures thereof in all ratios, and its physiologically tolerated salts.

2. A compound of the formula I as claimed in claim 1, in which
W is R¹-A-C(R¹³) and therein A is the divalent phenylene radical;
Y is a carbonyl group;
Z is N(R⁰);
B is a divalent methylene radical which is unsubstituted or is substituted by a (C₁-C₆)-alkyl radical;
E is R¹⁰CO;
R and R^{b} are independently of one another hydrogen or methyl;
R⁰ is (C₆-C₁₂)-aryl-(C₁-C₂)-alkyl optionally substituted in the aryl radical;
R¹ is H₂N-C(=NH), H₂N-C(=NOH), CH₃O-CO-NH-C(=NH), H₂N-C(=NH)-NH or H₂N-CH₂;
R², R^{2a} and R^{2b} are hydrogen;
R³ is R¹¹NH or CO-R⁵-Het
R⁵ is the divalent radical of a natural or unnatural α-amino acid having a lipophilic side chain, where free functional groups may be protected by protective groups customary in peptide chemistry or may be in the form of esters or amides, and where the nitrogen atom of the N-terminal amino group carries a radical R^{b};
R¹⁰ is hydroxy, (C₁-C₄)-alkoxy, phenoxy, benzyloxy or (C₁-C₄)-alkylcarbonyloxy-(C₁-C₄)-alkoxy;
R¹¹ is R¹⁵O-CO.
R¹³ is (C₁-C₆)-alkyl;
R¹⁵ is R¹⁶ or R¹⁶-CH₂;
R¹⁶ is cyclopentyl, cyclohexyl, 1-adamantyl, 2-adamantyl or noradamantyl;
Het is the radical of a 5-membered or 6-membered monocyclic heterocycle which is bonded via a ring nitrogen atom, which is saturated and which may optionally comprise an oxygen atom as additional ring heteroatom and which may be substituted by one or two identical or different (C₁-C₄)-alkyl radicals;
g and H are independently of one another 0 or 1;
in all its stereoisomeric forms and mixtures thereof in all ratios, and its physiologically tolerated salts.

3. A compound of the formula I as claimed in claim 1 and/or 2, in which R³ is R¹¹NH, g is 1 and h is 0, in all its stereoisomeric forms and mixtures thereof in all ratios, and its physiologically tolerated salts.

4. A compound of the formula I as claimed in one or more of claims 1 to 3, in which R¹⁶ is 1-adamantyl or 2-adamantyl, in all its stereoisomeric forms and mixtures thereof in all ratios, and its physiologically tolerated salts.

5. A compound of the formula I as claimed in claim 1 and/or 2, in which R³ is CO-R⁵-Het, g is 0 and h is 1, in all its stereoisomeric forms and mixtures thereof in all ratios, and its physiologically tolerated salts.

6. A compound of the formula I as claimed in one or more of claims 1 to 5, in which R¹³ is methyl, in all its stereoisomeric forms and mixtures thereof in all ratios, and its physiologically tolerated salts.

7. A process for preparing a compound of the formula I as claimed in one or more of claims 1 to 6, which comprises carrying out a fragment condensation of a compound of the formula II 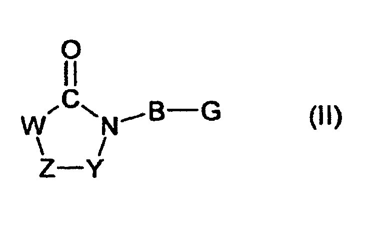 with a compound of the formula III 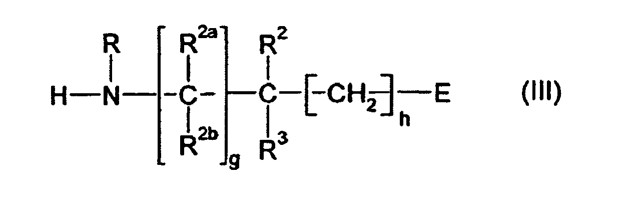 where the groups W, Y, Z, B, E, R, R², R^{2a}, R^{2b} and R³, and g and h in the formulae II and III are as defined in claims 1 to 6 or else functional groups may be present in protected form or in the form of precursors, and where G is hydroxycarbonyl, (C₁-C₆)-alkoxycarbonyl or an activated carboxylic acid derivative.

8. A compound of the formula I as claimed in one or more of claims 1 to 6 and/or its physiologically tolerated salts for use as medicament.

9. A pharmaceutical product which comprises one or more compounds of the formula I as claimed in one or more of claims 1 to 6 and/or their physiologically tolerated salts in addition to pharmaceutically acceptable carriers and/or additives.

10. A compound of the formula I as claimed in one or more of claims 1 to 6 and/or its physiologically tolerated salts for use as antiinflammatory agents.

11. A compound of the formula I as claimed in one or more of claims 1 to 6 and/or its physiologically tolerated salts for use in the therapy or prophylaxis of rheumatoid arthritis, of inflammatory bowel disease, of systemic lupus erythematosus or of inflammatory disorders of the central nervous system.

12. A compound of the formula I as claimed in one or more of claims 1 to 6 and/or its physiologically tolerated salts for use in the therapy or prophylaxis of asthma or allergies.

13. A compound of the formula I as claimed in one or more of claims 1 to 6 and/or its physiologically tolerated salts for use in the therapy or prophylaxis of cardiovascular disorders, of arteriosclerosis, of restenoses or of diabetes, for preventing damage of organ transplants, for inhibiting tumor growth or tumor metastasis or for therapy of malaria.

14. A compound of the formula I as claimed in one or more of claims 1 to 6 and/or its physiologically tolerated salts for use as inhibitors of the adhesion and/or migration of leukocytes or as inhibitors of the VLA-4 receptor.

## Revendications

1. Composé de formule I, 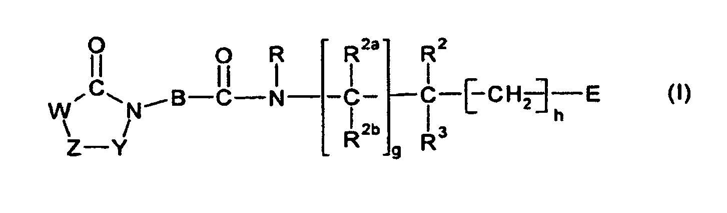 dans laquelle
W représente un groupe R¹-A-C(R¹³) et dans celui-ci, A représente un radical phénylène bivalent ;
Y représente un groupe carbonyle;
Z représente un groupe N(R⁰);
B représente un radical méthylène bivalent, qui est non substitué ou est substitué par un radical alkyle en (C₁-C₆);
E représente un groupe R¹⁰CO;
R et R^{b} représentent, indépendamment l'un de l'autre, un atome d'hydrogène ou un groupe alkyle en (C₁-C₄);
R⁰ représente un groupe (C₆-C₁₂)-aryl-(C₁-C₂)-alkyle éventuellement substitué dans le radical aryle ;
R¹ représente un groupe H₂N-C(=NH), un groupe H₂N-C(=N-OH), un groupe CH₃O-CO-NH-C(=NH), un groupe H₂N-C(=NH)-NH ou un groupe H₂N-CH₂;
R², R^{2a} et R^{2b} représentent un atome d'hydrogène ;
R³ représente un groupe R¹¹NH ou un groupe CO-R⁵-Het;
R⁵ représente le radical bivalent d'un α-amino-acide naturel ou non naturel présentant une chaîne latérale lipophile, où des groupes fonctionnels libres peuvent être protégés par des groupes protecteurs habituels dans la chimie peptidique, ou peuvent être présents sous la forme d'esters ou d'amides, et où l'atome d'azote du groupe amino N-terminal porte un radical R^{b};
R¹⁰ représente un groupe hydroxy, un groupe alcoxy en (C₁-C₄), un groupe phénoxy, un groupe benzyloxy ou un groupe (C₁-C₄)-alkylcarbonyloxy-(C₁-C₄)-alcoxy ;
R¹¹ représente un groupe R¹⁵O-CO ou un groupe R¹⁵S(O)₂;
R¹³ représente un groupe alkyle en (C₁-C₆);
R¹⁵ représente un groupe R¹⁶-(C₁-C₃)-alkyle ou un groupe R¹⁶;
R¹⁶ représente le radical d'un noyau monocyclique à 5-6 chaînons ou le radical d'un noyau bicyclique ou tricyclique à 7-12 chaînons, où ces noyaux sont saturés et peuvent également être substitués par un ou deux radicaux alkyle en (C₁-C₄) identiques ou différents ;
Het représente le radical d'un hétérocycle monocyclique à 5-6 chaînons, lié par l'intermédiaire d'un atome d'azote du noyau, qui est saturé et peut éventuellement renfermer un hétéroatome de noyau supplémentaire parmi le groupe constitué d'un atome d'oxygène et d'un atome de soufre, et qui peut être substitué par un ou deux radicaux alkyle en (C₁-C₄) identiques ou différents ;
g et h valent, indépendamment l'un de l'autre, 0 ou 1 ;
sous toutes ses formes stéréoisomères et leurs mélanges en tous rapports, et ses sels physiologiquement acceptables.

2. Composé de formule I selon la revendication 1, dans laquelle
W représente un groupe R¹-A-C(R¹³) et dans celui-ci, A représente un radical phénylène bivalent ;
Y représente un groupe carbonyle ;
Z représente un groupe N(R⁰) ;
B représente un radical méthylène bivalent, qui est non substitué ou est substitué par un radical alkyle en (C₁-C₆) ;
E représente un groupe R¹⁰CO ;
R et R^{b} représentent, indépendamment l'un de l'autre, un atome d'hydrogène ou un groupe méthyle ;
R⁰ représente un groupe (C₆-C₁₂)-aryl-(C₁-C₂)-alkyle éventuellement substitué dans le radical aryle ;
R¹ représente un groupe H₂N-C(=NH), un groupe H₂N-C(=NOH), un groupe CH₃O-CO-NH-C(=NH), un groupe H₂N-C(=NH)-NH ou un groupe H₂N-CH₂ ;
R², R^{2a} et R^{2b} représentent un atome d'hydrogène ;
R³ représente un groupe R¹¹NH ou un groupe CO-R⁵-Het ;
R⁵ représente le radical bivalent d'un α-amino-acide naturel ou non naturel présentant une chaîne latérale lipophile, où des groupes fonctionnels libres peuvent être protégés par des groupes protecteurs habituels dans la chimie peptidique, ou peuvent être présents sous la forme d'esters ou d'amides, et où l'atome d'azote du groupe amino N-terminal porte un radical R^{b} ;
R¹⁰ représente un groupe hydroxy, un groupe alcoxy en (C₁-C₄), un groupe phénoxy, un groupe benzyloxy ou un groupe (C₁-C₄)-alkylcarbonyloxy-(C₁-C₄)-alcoxy ;
R¹¹ représente un groupe R⁵O-CO ;
R¹³ représente un groupe alkyle en (C₁-C₆);
R¹⁵ représente un groupe R¹⁶ ou un groupe R¹⁶-CH₂ ;
R¹⁶ représente un groupe cyclopentyle, un groupe cyclohexyle, un groupe 1-adamantyle, un groupe 2-adamantyle ou un groupe noradamantyle ;
Het représente le radical d'un hétérocycle monocyclique à 5 ou 6 chaînons, lié par l'intermédiaire d'un atome d'azote du noyau, qui est saturé et peut éventuellement renfermer un atome d'oxygène en tant qu'hétéroatome de noyau supplémentaire, et qui peut être substitué par un ou deux radicaux alkyle en (C₁-C₄) identiques ou différents ;
g et h valent, indépendamment l'un de l'autre, 0 ou 1 ;
sous toutes ses formes stéréoisomères et leurs mélanges en tous rapports, et ses sels physiologiquement acceptables.

3. Composé de formule I selon la revendication 1 et/ou 2, dans laquelle R³ représente un groupe R¹¹NH, g vaut 1 et h vaut 0, sous toutes ses formes stéréoisomères et leurs mélanges en tous rapports, et ses sels physiologiquement acceptables.

4. Composé de formule I selon l'une ou plusieurs des revendications 1 à 3, dans laquelle R¹⁶ représente un groupe 1-adamantyle ou un groupe 2-adamantyle, sous toutes ses formes stéréoisomères et leurs mélanges en tous rapports, et ses sels physiologiquement acceptables.

5. Composé de formule I selon la revendication 1 et/ou 2, dans laquelle R³ représente un groupe CO-R⁵-Het, g vaut 0 et h vaut 1, sous toutes ses formes stéréoisomères et leurs mélanges en tous rapports, et ses sels physiologiquement acceptables.

6. Composé de formule I selon l'une ou plusieurs des revendications 1 à 5, dans laquelle R¹³ représente un groupe méthyle, sous toutes ses formes stéréoisomères et leurs mélanges en tous rapports, et ses sels physiologiquement acceptables.

7. Procédé de préparation d'un composé de formule I selon l'une ou plusieurs des revendications 1 à 6, **caractérisé en ce que** l'on réalise une condensation de fragments d'un composé de formule II 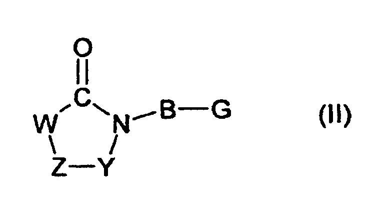 avec un composé de formule III 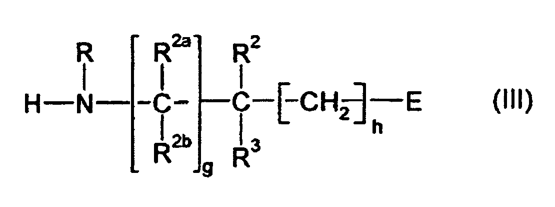 où, dans les formules II et III, les groupes W, Y, Z, B, E, R, R², R^{2a}, R^{2b} et R³ ainsi que g et h sont tels que définis dans les revendications 1 à 6, ou des groupes fonctionnels peuvent également être présents sous une forme protégée ou sous la forme de précurseurs, et où G représente un groupe hydroxycarbonyle, un groupe (C₁-C₆)-alcoxycarbonyle ou un dérivé d'acide carboxylique activé.

8. Composé de formule I selon l'une ou plusieurs des revendications 1 à 6 et/ou ses sels physiologiquement acceptables pour l'utilisation en tant qu'agent pharmaceutique.

9. Préparation pharmaceutique, **caractérisée en ce qu'**elle comprend un ou plusieurs composés de formule I selon l'une ou plusieurs des revendications 1 à 6 et/ou leurs sels physiologiquement acceptables, en plus de supports et/ou d'additifs irréprochables du point de vue pharmaceutique.

10. Composé de formule I selon l'une ou plusieurs des revendications 1 à 6 et/ou ses sels physiologiquement acceptables pour l'utilisation en tant qu'inhibiteurs d'inflammation.

11. Composé de formule I selon l'une ou plusieurs des revendications 1 à 6 et/ou ses sels physiologiquement acceptables pour l'utilisation dans la thérapie ou la prophylaxie de la polyarthrite rhumatoïde, de la maladie intestinale inflammatoire, du lupus érythémateux disséminé ou de troubles inflammatoires du système nerveux central.

12. Composé de formule I selon l'une ou plusieurs des revendications 1 à 6 et/ou ses sels physiologiquement acceptables pour l'utilisation dans la thérapie ou la prophylaxie de l'asthme ou d'allergies.

13. Composé de formule I selon l'une ou plusieurs des revendications 1 à 6 et/ou ses sels physiologiquement acceptables pour l'utilisation dans la thérapie ou la prophylaxie de troubles cardiovasculaires, de l'artériosclérose, de resténoses ou du diabète, pour empêcher l'endommagement de greffes d'organe, pour l'inhibition d'une croissance tumorale ou d'une métastase tumorale, ou pour la thérapie de la malaria.

14. Composé de formule I selon l'une ou plusieurs des revendications 1 à 6 et/ou ses sels physiologiquement acceptables pour l'utilisation en tant qu'inhibiteurs de l'adhésion et/ou de la migration de leucocytes ou en tant qu'inhibiteurs du récepteur VLA-4.
